(19) Europäisches Patentamt
European Patent Office
Office européen des brevets

(11) Publication number: **0 161 877 B1**

(12) # EUROPEAN PATENT SPECIFICATION

(45) Date of publication of patent specification: **29.05.91**

(51) Int. Cl.5: **C07D 211/90**, C07D 405/12, C07D 409/12, C07D 401/12, C07D 401/04, C07D 409/04, C07D 405/04, A61K 31/44

(21) Application number: **85303141.7**

(22) Date of filing: **02.05.85**

(54) **1,4-dihydropyridine derivatives, methods for their production and pharmaceutical compositions comprising the same.**

(30) Priority: **04.05.84 JP 88411/84**
**20.06.84 JP 125379/84**

(43) Date of publication of application:
**21.11.85 Bulletin 85/47**

(45) Publication of the grant of the patent:
**29.05.91 Bulletin 91/22**

(84) Designated Contracting States:
**CH DE FR GB IT LI NL**

(56) References cited:
**EP-A- 0 042 566**
**EP-A- 0 052 300**
**EP-A- 0 063 359**
**EP-A- 0 063 747**
**EP-A- 0 088 903**
**EP-A- 0 145 434**
**EP-A- 0 173 126**
**DE-A- 2 005 116**
**DE-A- 2 921 429**

(73) Proprietor: **FUJIREBIO KABUSHIKI KAISHA also trading as FUJIREBIO INC.**
**6-7, Shimoochiai 4-chome Shinjuku-ku**
**Tokyo 161(JP)**

(72) Inventor: **Kutsuma, Teruo**
**442-15, Hireao**
**Inagi-shi Tokyo(JP)**
Inventor: **Ikawa, Hiroshi**
**26-10, Miyasaka 1-chome**
**Setagaya-ku Tokyo(JP)**
Inventor: **Sato, Yoshiaki**
**32-4, Asahigaoka 2-chome**
**Hino-shi Tokyo(JP)**

(74) Representative: **Silverman, Warren et al**
**HASELTINE LAKE & CO. Hazlitt House 28**
**Southampton Buildings Chancery Lane**
**London WC2A 1AT(GB)**

EP 0 161 877 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel 1,4-dihydropyridine derivatives having hypotensive action.

It is known that some 1,4-dihydropyridine derivatives have hypotensive action. For example, 4-(o-nitrophenyl)-2,6-dimethyl-1,4-dihydropyridine-3,5-dicarboxylic acid dimethyl ester (US-A-3,644,627; hereinafter referred to as "nifedipine") and 4-(m-nitrophenyl)-2,6-dimethyl-1,4-dihydropyridine-3,5-dicarboxylic acid-3-methyl ester-5-[2-(benzylmethylamino)-ethyl] ester hydrochloride (Japanese Patent Publication No.45075/1980; hereinafter referred to as "nicardipine") have already been utilized in medicine for their hypotensive action and coronary vasodilation effect. Moreover, it is also known that many compounds similar to nifedipine and nicardipine and represented by the following general formula also have hypotensive action (Japanese Patent Publication No.29989/1980, ibid No.29990/1980, Japanese Patent Application Kokai No.64571/1980, etc.).

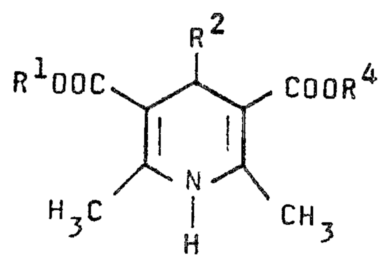

in which $R^1$ is an alkyl group, for example methyl or ethyl, $R^2$ is, inter alia, 3-nitrophenyl, and $R^4$ is alkyl, for example ethyl or isopropyl, alkenyl, for example allyl, or alkynyl, for example propargyl.

However, these compounds have the disadvantage that the time their effect is maintained (effecting time) is short. For example, it has been reported that when 10 μg/kg of nicardipine whose effecting time is longer than that of nifedipine, were injected into a vein of a dog, the effecting time was only about 30 to 40 minutes (Arzneim-Forsch, vol. 22, p 33 (1976), ibid., vol. 26, p 2172 (1982), Tohoigakkai Zasshi, vol. 26, No.2, p 48 (1972)).

Generally, when treating hypertension it is desirable for the effecting time to be long so that the lowering of blood pressure takes place slowly.

It is an object of the invention to provide a compound having hypotensive action whose effecting time is long and which only reduces blood pressure slowly.

The 1,4-dihydropyridine derivatives of the invention is characterized by the replacement of $R^4$ such as ethyl group, isopropyl group, allyl group, propargyl group, etc. in the aforesaid general formula by a combination group of an unsaturated straight chain hydrocarbyl group or derivative thereof and an unsaturated hydrocarbyl group or an aryl or heteroaromatic group linked to an unsaturated carbon chain of the straight chain hydrocarbon..

According to one aspect of this invention, there are provided 1,4-dihydropyridine derivatives represented by the general formula

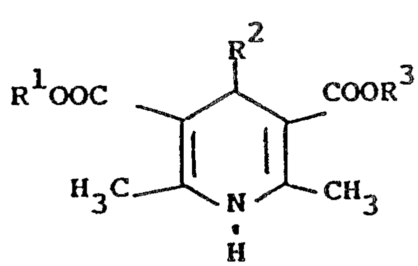

in which

$R^1$ represents a saturated or unsaturated, straight-chain, branched or cyclic hydrocarbyl group containing up to 6 carbon atoms, in which one carbon atom of the carbon skeleton thereof is optionally replaced by an oxygen or sulphur atom and/or in which one or more hydrogen atoms is/are optionally substituted by phenyl, phenoxy or phenylthio;

$R^2$ represents phenyl, naphthyl or furyl in which one or more hydrogen atoms is/are optionally substituted

by a mciety selected from halogen atoms, alkylthio groups, nitro groups and cyano groups, and

$R^3$ represents (A), with $R^2$ being phenyl or substituted phenyl, a straight-chain or branched hydrocarbyl group containing two double bonds conjugated with each other, or (B) a group which is (a) an unsaturated straight-chain or branched aliphatic hydrocarbyl group containing from 3 to 6 carbon atoms, which group is optionally substituted by phenyl, an unsaturated carbon atom of said hydrocarbyl group having attached thereto a ring carbon atom of (b) a phenyl, naphthyl, furyl, thienyl, N-methyl-1-pyrrolyl or pyridyl group, with a said phenyl group optionally being substituted by a group selected from methyl, nitro and cyano groups, excluding a compound which is cinnamyl methyl 4-(3-nitrophenyl)-2,6-dimethyl-1,4-dihydropyridine-3,5-dicarboxylate or methyl phenylpropargyl 4-(3-nitrophenyl)-2,6-dimethyl-1,4-dihydropyridine-3,5-dicarboxylate.

According to a second aspect of this invention, there are provided 1,4-dihydropyridine derivatives represented by the general formula

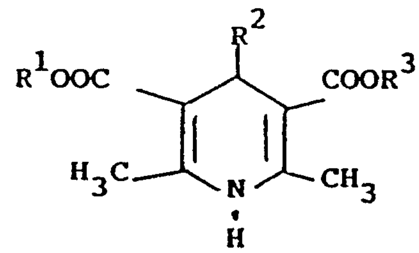

in which

$R^1$ represents a saturated or unsaturated, straight-chain, branched or cyclic hydrocarbyl group containing up to 6 carbon atoms or an analogue thereof in which one or more carbon atoms of the carbon skeleton thereof is replaced by an oxygen atom or a sulphur atom and/or in which one or more hydrogen atoms is/are substituted by moieties selected from phenyl, phenoxy and phenylthio;

$R^2$ represents a phenyl, naphthyl, furyl,pyridyl or thienyl group in which one or more hydrogen atoms is/are optionally substituted by a moiety selected from halogen atoms, alkylthio groups, nitro groups and cyano groups, and

$R^3$ represents either (A) a group which is (a) an unsaturated straight-chain hydrocarbyl group containing 3 to 6 carbon atoms and in which a carbon atom of an unsaturated group is essentially bound by a direct chemical bond to an unsaturated carbon atom of (b) an unsaturated hydrocarbyl group containing up to 6 carbon atoms such that an unsaturated double bond of (a) is in conjunction with an unsaturated double bond of (b), or (B) a group which is (c) a said unsaturated straight-chain hydrocarbyl group (a) or a said unsaturated straight-chain hydrocarbyl group (a) which is phenyl substituted or in which a hydrogen atom at the 1-position is substituted by $C_{1-4}$ alkyl or both hydrogen atoms at the 1-position are substituted by methyl groups, an unsaturated carbon atom of said unsaturated straight-chain hydrocarbyl group having attached thereto a ring carbon atom of (d) a phenyl, naphthyl, furyl, thienyl, N-methyl-2-pyrrolyl or pyridyl group, with one or more hydrogen atoms of a said phenyl group being optionally substituted by a moiety selected from methyl, cyano and nitro groups,

excluding a compound which is cinnamyl methyl 4-(3-nitrophenyl)-2,6-dimethyl-1,4-dihydropyridine-3,5-dicarboxylate or methyl phenylpropargyl (or 2-thienylpropargyl or 2-pyridylpropargyl) 4-(3-nitrophenyl)-2,6-dimethyl-1,4-dihydropyridine-3,5-dicarboxylate.

This invention also includes pharmaceutically acceptable acid addition salts of compounds as aforesaid.

EP-A-145434 published on 19 June 1985 disclosed 1,4-dihydropyridine derivatives having, inter alia, hypotensive action. Having regard to Article 54(3) EPC we exclude from our invention various groups of compounds according to the foregoing provisoes.

Insofar as the second aspect of the invention is concerned, and also the first aspect of the invention, insofar as the restrictions of the definitions therein permit, the hydrocarbyl group $R^1$ may be saturated or unsaturated, and may be straight chain, branched or cyclic and contains up to 6 carbon atoms. Such hydrocarbyl groups include methyl, ethyl, n-propyl, isopropyl, n-butyl, isobutyl, tert.butyl, n-pentyl, n-hexyl, allyl, 3-butenyl, crotyl, 3-butene-2-yl, 2-butynyl, 3-butynyl, cyclohexyl, 2-pentyl, 3-pentyl, 4-penten-1-yl, 3-penten-2-yl, 2-penten-1-yl, 2,4-hexadienyl, 2-hexen-1-yl, 1-hexen-3-yl, propargyl, cyclopropylmethyl, 3-pentynyl, 2-hexen-3-yn-1-yl and 2-cyclohexenyl groups. One or mare carbon atoms of the skeleton of the hydrocarbyl group may be replaced by an oxygen atom or a sulphur atom to produce a hydrocarbyl group analogue. Preferably, one or more carbon atoms of a carbon chain or a carbocyclic ring is/are replaced by

oxygen atom(s) and/or one or more hydrogen atoms is/are substituted by phenyl. Such a hydrocarbyl group analogue is exemplified by 2-methoxyethyl, 2-ethoxyethyl, 2-(2-methoxyethoxy)ethyl, 2-butoxyethyl, 2-(2-ethoxyethoxy)ethyl, 3-methoxybutyl, 4-methoxy-3-butene-2-yl, 2-(2-butoxyethoxy)ethyl, 2-cyclohexyloxyethyl, 2-methyithioethyl, 2-ethylthioethyl, 3-methylthiobutyl and 2-cyclohexylthioethyl groups. One or more hydrogen atoms of the hydrocarbon group or its analogue mentioned above may be substituted by phenyl, phenoxy or phenylthio.

$R^2$ is an aryl or heteroaromatic group, specifically selected from phenyl, thienyl, furyl, pyridyl, and naphthyl groups. One or more hydrogen atoms of the aryl group or the heteroaromatic group may be substituted by halogen, alkylthio, nitro or cyano. The halogen atom may be chlorine, bromine, iodine or fluorine.

$R^3$ is a combination of an unsaturated straight chain hydrocarbyl group or derivative thereof and an unsaturated hydrocarbyl group. The unsaturated straight chain hydrocarbyl group is, for example 1-propen-1-yl, 2-butene-2-yl, 1-butene-1-yl, 1-penten-1-yl, 3-penten-3-yl, 2-penten-2-yl, 1-propyn-1-yl, 2-butyn-1-yl, 1,3-hexadienyl-, 1-hexen-1-yl, 3-hexen-3-yl, or 1-hexen-2-yn-1-yl. One or more hydrogen atoms at the 1-position may be substituted by $C_{1-4}$ alkyl, for example methyl, ethyl, n-propyl, i-propyl, n-butyl, t-butyl or phenyl.

The unsaturated hydrocarbyl group branched off from the unsaturated straight chain hydrocarbyl group is, for example, allyl, 2-butene-1-yl, 3-butene-1-yl, -buten-2-yl, 2-penten-1-yl, 3-penten-1-yl, 4-penten-1-yl, 3-penten-2-yl, propargyl, 3-butyn-1-yl, 2-butyn-1-yl, 2,4-hexadienyl, 2-hexen-3-yl, 3-hexen-1-yl, 3-pentynyl, or 2-hexen-2-yn-1-yl.

A carbon atom of an unsaturated group of the above hydrocarbyl group is connected to a carbon atom of an unsaturated group of the above unsaturated straight chain hydrocarbon or derivative thereof, by a direct chemical linkage. As the result, the two unsaturated bonds connected together are conjugated. The term multiple bond as used herein means double, single and (see last paragraph), hybridised bonds of aromatic or heteroaromatic rings.

Alternatively, $R^3$ is a combination of an unsaturated straight chain hydrocarbyl group or derivative thereof and a phenyl, naphthyl, cyclohexenyl, furyl, thienyl, N-methylpyrrolyl or pyridyl group in which one or more hydrogen atoms is/are optionally substituted by a moiety selected from methyl, nitro and cyano and in which a ring carbon atom is connected to a carbon atom of an unsaturated group of the unsaturated straight chain hydrocarbon or derivative thereof so as to provide conjugation of multiple bonds.

Geometrical isomers of the 1,4-dihydropyridine derivatives which can exist are considered to lie within the ambit of this invention.

The 1,4-dihydropyridine derivatives of this invention display hypotensive action. When 1 mg/kg of such a derivative suspended in gum arabic solution in a concentration of 5% by weight is injected into the instinum duodenum of a rat of more than 10 week growth exhibiting spontaneous hypertension, there is a drop in the blood pressure by more than 20 mmHg, usually more than 35 mmHg. The time to reach the lowest blood pressure is more than 20 minutes, usually more than 40 minutes. The half-life period of the activity of the 1,4-dihydropyridine derivative is more than 60 minutes, usually more than 120 minutes. Both the cis isomer and the trans isomer have hypotensive action.

This invention also provides methods for the production of the aforesaid 1,4-dihydropyridine derivatives of this invention which may be listed as follows:

<u>Method 1</u>

A compound having the following general formula

$$CH_3-\overset{O}{\overset{\|}{C}}-CH_2-\overset{O}{\overset{\|}{C}}-OR^3$$

a compound having the following general formula

$R^2$-CHO

and a compound having the following general formula

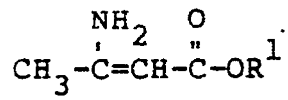

are mixed with each other in the absence of solvent or in a solvent inert to the reaction, for example methanol, ethanol, propanol, isopropanol, benzene, toluene, dioxane, tetrahydrofuran, dimethyl sulphoxide or dimethylformamide, and the reaction mixture is heated to produce the derivative of the invention. The preferred reaction temperature is from 50 to 150° C, and the reaction time is usually from 0.5 to 15 hours.

Method 2

By following a procedure analogous to that used in Method 1, one may react with a compound of general formula

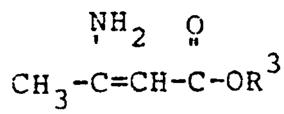

a compound having the following general formula

$R^2$-CHO

and a compound having the following general formula

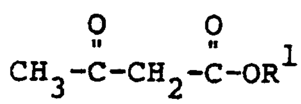

Method 3

Compounds having the general formulae

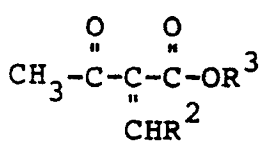 and 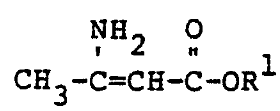

are reacted together under the conditions indicated in Method 1.

Method 4

Compounds having the general formulae

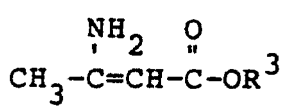 and 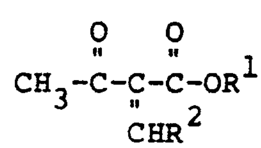

are reacted together under the conditions indicated in Method 1.

Method 5

Ammonia and compounds having the general formulae

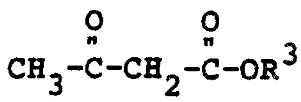

and

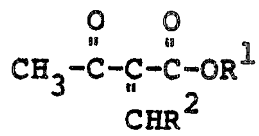

are reacted together under the conditions indicated in Method 1.

Method 6

Ammonia and compounds having the general formulae

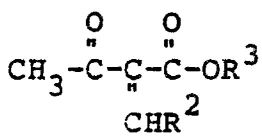

and

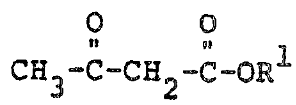

are reacted together under the conditions indicated in Method 1.

Method 7

A compound having the following general formula

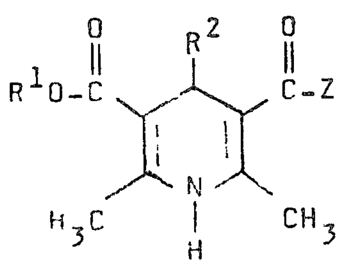

in which Z represents a hydroxyl group or an acid residue of an active ester, for example a halogen atom, a methylsulphonyloxy group, paratoluenesulphonyloxy group or benzotriazole-1-oxy group
and an alcohol having the following formula

$R^3$-OH

are allowed to react together to produce a 1,4-dihydropyridine derivative of the invention. When Z is a hydroxyl group, the reaction may be carried out in the presence of an acid such as hydrogen chloride, sulphuric acid or boron trifluoride or a dehydrating condensing agent, for example dicyclohexylcarbodiimide, in an inert solvent. A base, for example 4-dimethylaminopyridine may be present in the reaction mixture. When Z is an active ester residue, the reaction may be carried out in an inert solvent, optionally in the presence of a base such as triethylamine, 4-dimethylaminopyridine, pyridine or potassium carbonate.

Method 8

A compound of the general formula

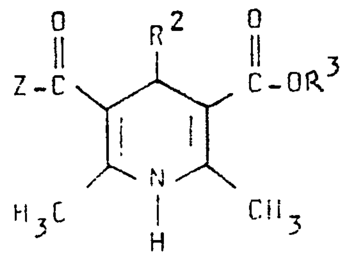

and an alcohol of the general formula $R^1OH$ are allowed to react in the same manner as in Method 7 to produce a 1,4-dihydropyridine derivative of the invention.

The 1,4-dihydropyridine derivatives so produced may be separated from the reaction mixture and purified by conventional procedures.

The 1,4-dihydropyridine derivatives of the invention have strongly vasodilative and hypotensive actions. The time to reach the lowest blood pressure is much later than conventionally, and the half-life period of the hypotensive activity of the compounds is sufficiently long. These derivatives are thus useful drugs for the treatment of hypertension. Hence this invention also provides a pharmaceutical composition which comprises a compound of this invention in association with a pharmacologically acceptable adjuvant.

The following Examples illustrate the invention:

Example 1

3.52 g (10 mM) of 2-(4-methylthiobenzylidene) acetoacetic acid cinnamyl ester were mixed with 1.38 g (12 mM) of 3-aminocrotonic acid methyl ester, and heated at 120°C for 3 hours. The reaction mixture was separated by silica gel column chromatography, and cinnamyl methyl 4-(4-methylthiophenyl)-2,6-dimethyl-1,4-dihydropyridine-3,5-dicarboxylate was obtained in satisfactory yield. This derivative was recrystallized once from methanol.

Elemental Analysis; $C_{26}H_{27}NO_4S$

Calcd. (%) C: 69.46, H: 6.05; N: 3.12

Found (%) C: 69.51, H: 5.94, N: 3.07

m.p.; 164.2-170.8°C

IR ($cm^{-1}$); $\nu_{NH}$ 3330, $\nu_{CO}$ 1680

NMR $\delta_{CDCl_3}$; 2.26(s,3H), 2.28(s,3H), 2.35(s,3H), 3.61(s,3H), 4.0-4.82(m,2H), 5.00(s,1H), 5.87-6.69 (m,2H), 6.17(s,1H), 6.7-7.8(m9H)

The following 1,4-dihydropyridine derivatives were prepared by following the same procedure as set out in Example 1 but using different starting compounds. When an oily product was produced, recrystallization was not carried out.

Example 2

Cinnamyl methyl 4-(2-cyanophenyl)-2,6-dimethyl-1,4-dihydropyridine-3,5-dicarboxylate

Elemental Analysis; $C_{26}H_{24}N_2O_4$

Calcd. (%) C: 72.71, H: 5.87, N: 6.52

Found (%) C: 72.75, H: 5.79, N: 6.48

m.p.; Oily

IR ($cm^{-1}$); $\nu_{NH}$ 3330, $\nu_{CN}$ 2230, $\nu_{CO}$ 1700

NMR $\delta_{CDCl_3}$; 2.32(s,6H), 3.61(s,3H), 4.5-4.9 (m,2H), 5.35(s1H), 5.9-6.3 (m,2H), 6.74 (s,1H), 6.9-7.6(m,9H)

Example 3

1-tert.butyl-3-phenyl-2-propenyl methyl 4-(3-nitrophenyl)-2,6-dimethyl-1,4-dihydropyridine-3,5-dicarboxylate

Elemental Analysis; $C_{29}H_{32}N_2O_6$

Calcd. (%) C: 69.03, H: 6.39, N: 5.55

Found (%) C: 69.06, H: 6.43, N: 5.51

m.p.; 166.5-168°C

IR ($cm^{-1}$); $\nu_{NH}$ 3340, $\nu_{CO}$ 1700, 1670

$\nu_{NO_2}$ 1530, 1350

NMR $\delta_{CDCl_3}$; 1.00(s,9H), 2.34(s,6H), 3.64(s,3H), 5.10(m,1H), 5.18(s,1H), 5.9-6.6(m,3H), 7.1-8.2(m,9H)

Example 4

3-(2-furyl)-2-propenyl methyl 4-(3-nitrophenyl)-2,6-dimethyl-1,4-dihydropyridine-3,5-dicarboxylate

Elemental Analysis; $C_{23}H_{22}N_2O_7$

Calcd. (%) C: 63.01, H: 5.06, N: 6.39

Found (%) C: 63.05, H: 5.01, N: 6.36

m.p.; 157-159.5°C

IR ($cm^{-1}$); $\nu_{NH}$ 3320, $\nu_{CO}$ 1705, 1655

$\nu_{NO_2}$ 1530, 1350

NMR $\delta_{CDCl_3}$; 2.34(s,6H), 3.62(s,3H), 4.64(d,2H), 5.12(s,1H), 6.20(m,5H), 7.1-8.1(m,5H)

Example 5

1-isopropyl-3-phenyl-2-propenyl methyl 4-(3-nitrophenyl)-2-6-dimethyl-1,4-dihydropyridine-3,5-dicarboxylate

Elemental Analysis; $C_{28}H_{30}N_2O_6$

Calcd. (%) C: 68.56, H: 6.16, N: 5.71

Found (%) C: 68.58, H: 6.13, N: 5.69

m.p.; Oily

IR ($cm^{-1}$); $\nu_{NH}$ 3320, $\nu_{CO}$ 1680, $\nu_{NO_2}$ 1530, 1350

NMR $\delta_{CDCl_3}$; 1.00(d,6H), 2.36(s,6H), 3.64(s,3H), 5.16(s,1H), 5.18(m,1H), 5.9-6.8(m,3H), 7.0-8.2(m,9H)

Example 6

3,3-diphenyl-2-propenyl methyl 4-(3-nitrophenyl)-2,6-dimethyl-1,4-dihydropyridine-3,5-dicarboxylate

Elemental Analysis; $C_{31}H_{28}N_2O_6$

Calcd. (%) C: 70.97, H: 5.38, N: 5.34

Found (%) C: 71.04, H: 5.32, N: 5.28

m.p.; Oily

IR ($cm^{-1}$); $\nu_{NH}$ 3330, $\nu_{CO}$ 1705, $\nu_{NO_2}$ 1530, 1350

NMR $\delta_{CDCl_3}$; 2.30(s,6H), 3.61(s,3H), 4.57(d,2H), 5.10(s,1H), 5.90(d,1H), 6.18(s,1H), 6.9-8.05(m,14H)

Example 7

1-propyl-3-phenyl-2-propenyl methyl 4-(3-nitrophenyl)-2,6-dimethyl-1,4-dihydropyridine-3,5-dicarboxylate

Elemental Analysis; $C_{28}H_{30}N_2O_6$

Calcd. (%) C: 68.56, H: 6.16, N: 5.71

Found (%) C: 68.61, H: 6.12, N: 5.68

m.p.; Oily

IR ($cm^{-1}$); $\nu_{NH}$ 3330, $\nu_{CO}$ 1680, $\nu_{NO_2}$ 1530, 1350

NMR $\delta_{CDCl_3}$; 0.7-2.0(m,7H), 2.32(s,6H), 3.60(s,3H), 5.10(s,1H), 5.35(m,1H), 5.9-6.8(m,3H), 7.1-8.2(m,9H)

Example 8

4-α-thienyl-3-butenyl methyl 4-(3-nitrophenyl)-2,6-dimethyl-1,4-dihydropyridine-3,5-dicarboxylate

Elemental Analysis; $C_{24}H_{24}N_2O_6S$

Calcd. (%) C: 61.53, H: 5.16, N: 5.98

Found (%) C: 61.57, H: 5.13, N: 5.79

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 2.33(s,6H), 2.52(t,2H), 3.58(s,3H), 4.16(t,3H), 5.11(s,1H), 5.5-8.2(m,10H)

Example 9

4-β-naphthyl-3-butenyl methyl 4-(3-nitrophenyl)-2,6-dimethyl-1,4-dihydropyridine-3,5-dicarboxylate

Elemental Analysis; $C_{30}H_{28}N_2O_6$

Calcd. (%) C: 70.30, H: 5.51, N: 5.47

Found (%) C: 70.34, H: 5.47, N: 5.42

(trans)

m.p.; 180.5-181.5°C

IR ($cm^{-1}$); $\nu_{NH}$ 3370, $\nu_{CO}$ 1700, 1660, $\nu_{NO_2}$ 1535, 1355

NMR $\delta_{CDCl_3}$; 2.26(s,6H), 2.52(m,2H), 3.52(s,3H), 4.20(t,2H), 5.10(s,1H), 6.10(tt,1H), 6.25(bs,1H), 6.54(d,1H), 7.0-8.1(m,11H)

(cis)

m.p.; 139-140°C

IR ($cm^{-1}$); $\nu_{NH}$ 3360, $\nu_{CO}$ 1710, 1650, $\nu_{NO_2}$ 1530, 1350

NMR $\delta_{CDCl_3}$; 2.28(s,6H), 2.75(m,2H), 3.56(s,3H), 4.14(t,2H), 5.05(s,1H), 5.60(tt,1H), 6.25(bs,1H), 6.60(d,1H), 7.1-8.2(m,11H)

Example 10

Cinnamyl 2-methoxyethyl 4-(3-nitrophenyl)-2,6-dimethyl-1,4-dihydropyridine-3,5-dicarboxylate

Elemental Analysis; $C_{27}H_{28}N_2O_7$

Calcd. (%) C: 65.84, H: 5.73, N: 5.69

Found (%) C: 65.88, H: 5.70, N: 5.66

m.p.; 115.5-116.5°C

IR ($cm^{-1}$); $\nu_{NH}$ 3380, $\nu_{CO}$ 1710, 1680, $\nu_{NO_2}$ 1530, 1350

NMR $\delta_{CDCl_3}$; 2.34(s,6H), 3.25(s,3H), 3.50(t,2H), 4.15(t,2H), 4.68(d,2H), 5.15(s,1H), 5.9-6.9(m,3H), 7.1-8.2(m,9H)

Example 11

4-(4-cyanophenyl)-3-butenyl methyl 4-(3-nitrophenyl)-2,6-dimethyl-1,4-dihydropyridine-3,5-dicarboxylate

Elemental Analysis; $C_{27}H_{25}N_3O_6$

Calcd. (%) C: 66.52, H: 5.17, N: 8.62

Found (%) C: 66.50, H: 5.19, N: 8.59

m.p.; 161.3-164.8°C

IR ($cm^{-1}$); $\nu_{NH}$ 3330, $\nu_{CO}$ 1690, $\nu_{NO_2}$ 1530, 1350

NMR $\delta_{CDCl_3}$; 2.35(s,6H), 2.58(t,2H), 3.58(s,3H), 4.20(t,2H), 5.08(s,1H), 6.15-6.40(m,3H), 7.2-8.1(m,8H)

Example 12

4-(N-methyl-2-pyrrolyl)-butenyl methyl 4-(3-nitrophenyl)-2,6-dimethyl-1,4-dihydropyridine-3,5-dicarboxylate

Elemental Analysis; $C_{25}H_{27}N_3O_6$

Calcd. (%) C: 64.51, H: 5.85, N: 9.03

Found (%) C: 64.58, H: 5.80, N: 8.97

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 2.30(s,6H), 2.60(m,2H), 3.53(s,3H), 3.60(s,3H), 4.15(t,2H), 5.06(s,1H), 5.1-6.6(m,6H), 7.1-8.1(m,4H)

Example 13

4-(p-methylphenyl)-3-butenyl methyl 4-(3-nitrophenyl)-2,6-dimethyl-1,4-dihydropyridine-3,5-dicarboxylate

Elemental Analysis; $C_{27}H_{28}N_2O_6$

Calcd. (%) C: 68.05, H: 5.92, N: 5.88

Found (%) C: 68.11, H: 5.83, N: 5.82

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 2.30(s,9H), 2.52(t,2H), 3.55(s,3H), 4.13(t,2H), 5.07(s,1H), 6.0-8.1(m,11H)

Example 14

4(p-nitropnenyl)-3-butenyl methyl 4-(3-nitrophenyl)-2,6-dimethyl-1,4-dihydropyridine-3,5-dicarboxylate

Elemental Analysis; $C_{26}H_{25}N_3O_8$

Calcd. (%) C: 61.53, H: 4.97, N: 8.28

Found (%) C: 61.59, H: 4.79, N: 8.16

m.p.; 111-113.5°C

IR ($cm^{-1}$); $\nu_{NH}$ 3340, $\nu_{CO}$ 1690, 1650, $\nu_{NO_2}$ 1525, 1350

NMR $\delta_{CDCl_3}$; 2.32(s,6H), 2.65(m,2H), 3.58(s,3H), 4.15(m,2H), 5.04(s,1H), 5.5-6.6(m,3H), 7.1-8.2(m,8H)

Example 15

1,1-dimethyl-3-phenyl-2-propenyl methyl 4-(3-nitrophenyl)-2,6-dimethyl-1,4-dihydropyridine-3,5-dicarboxylate

Elemental Analysis; $C_{27}H_{28}N_2O_6$

Calcd. (%) C: 68.05, H: 5.92, N: 5.88

Found (%) C: 68.10, H: 5.88, N: 5.83

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 1.60(d,6H), 2.30(s,6H), 3.51(s,3H), 5.05(s,1H), 5.98(s,1H), 6.30(s,2H), 7.1-8.2(m,9H)

Example 16

1-ethyl-3-phenyl-2-propenyl methyl 4-(3-nitrophenyl)-2,6-dimethyl-1,4-dihydropyridine-3,5-dicarboxylate

Elemental Analysis; $C_{27}H_{28}N_2O_6$

Calcd. (%) C: 68.05, H: 5.92, N: 5.88

Found (%) C: 68.09, H: 5.90, N: 5.85

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 1.70(t,9H), 1.70(q,2H), 2.30(d,6H), 3.59(s,3H), 5.09(s,1H), 5.30(t,1H), 5.8-6.6(m,3H), 7.0-8.1(m,9H)

Example 17

4-(3-thienyl)-3-butenyl methyl 4-(3-nitrophenyl)-2,6-dimethyl-1,4-dihydropyridine-3,5-dicarboxylate

Elemental Analysis; $C_{24}H_{24}N_2O_6S$

Calcd. (%) C: 61.53, H: 5.16, N: 5.98

Found (%) C: 61.55, H: 5.13, N: 5.89

m.p.; 122.1-127.7°C

IR ($cm^{-1}$); $\nu_{NH}$ 3340, $\nu_{CO}$ 1690, $\nu_{NO_2}$ 1530, 1350

NMR $\delta_{CDCl_3}$; 2.30(s,6H), 2.4-2.8(m,2H), 3.57(d,3H), 4.12(t,2H), 5.07(s,1H), 5.7-8.1(m,10H)

Example 18

1,3-diphenyl-2-propenyl methyl 4-(3-nitrophenyl)-2,6-dimethyl-1,4-dihydropyridine-3,5-dicarboxylate

Elemental Analysis; $C_{31}H_{28}N_2O_6$

Calcd. (%) C: 70.97, H: 5.38, N: 5.34

Found (%) C: 70.94, H: 5.44, N: 5.30

m.p.; 143-145.5°C

IR ($cm^{-1}$); $\nu_{NH}$ 3380, $\nu_{CO}$ 1695, $\nu_{NO_2}$ 1525, 1350

NMR $\delta_{CDCl_3}$; 2.62(s,6H), 3.60(s,3H), 5.15(s,1H), 6.2-6.5(m,4H), 7.0-8.2(m,14H)

Example 19

4-(2-pyridyl)-3-butenyl methyl 4-(3-nitrophenyl)-2,6-dimethyl-1,4-dihydropyridine-3,5-dicarboxylate

Elemental Analysis; $C_{25}H_{25}N_3O_6$

Calcd. (%) C: 64.79, H: 5.44, N: 9.07

Found (%) C: 64.85, H: 5.39, N: 9.06

m.p.; Oily

IR ($cm^{-1}$); $\nu_{NH}$ 3330, $\nu_{CO}$ 1700, $\nu_{NO_2}$ 1530, 1350

NMR $\delta_{CDCl_3}$; 2.31(s,6H), 2.60(t,2H), 3.57(s,3H), 4.20(t,2H), 5.09(s,1H), 6.4-8.6(m,9H)

Example 20

3-(2-naphthyl)-2-propenyl methyl 4-(3-nitrophenyl)-2,6-dimethyl-1,4-dihydropyridine-3,5-dicarboxylate

Elemental Analysis; $C_{29}H_{26}N_2O_6$

Calcd. (%) C: 69.87, H: 5.26, N: 5.62

Found (%) C: 69.99, H: 5.22, N: 5.57

m.p.; 149.4-154.3°C

IR ($cm^{-1}$); $\nu_{NH}$ 3330, $\nu_{CO}$ 1700, $\nu_{NO_2}$ 1530, 1350

NMR $\delta_{CDCl_3}$; 2.30(s,3H), 2.34(s,3H), 3.60(s,3H), 4.74(d,2H), 5.15(s,1H), 6.0-6.85(m,3H), 7.1-8.2(m,11H)

Example 21

1-methyl-3-phenyl-2-propenyl methyl 4-(3-nitrophenyl)-2,6-dimethyl-1,4-dihydropyridine-3,5-dicarboxylate

Elemental Analysis; $C_{26}H_{26}N_2O_6$

Calcd. (%) C: 67.52, H: 5.67, N: 6.06

Found (%) C: 67.71, H: 5.66, N: 6.01

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 1.35(m,3H), 2.30(s,6H), 3.60(s,3H), 5.12(s,1H), 5.45(m,1H), 6.0-6.8(m,3H), 7.1-8.2(m,9H)

Example 22

1-methyl-3-phenyl-2-propenyl ethyl 4-(3-nitrophenyl)-2,6-dimethyl-1,4-dihydropyridine-3,5-dicarboxylate

Elemental Analysis; $C_{27}H_{28}N_2O_6$

Calcd. (%) C: 68.05, H: 5.92, N: 5.88

Found (%) C: 68.11, H: 5.88, N: 5.83

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 1.25(d,3H), 1.26(q,3H), 2.30(s,6H), 4.05(q,2H), 5.08(s,1H), 5.37(q,1H), 5.9-6.7(m,3H), 7.0-8.15(m,9H)

Example 23

1-methyl-3-phenyl-2-propenyl isopropyl 4-(3-nitrophenyl)-2,6-dimethyl-1,4-dihydropyridine-3,5-dicarboxylate

Elemental Analysis; $C_{28}H_{30}N_2O_6$

Calcd. (%) C: 68.56, H: 6.16, N: 5.71

Found (%) C: 68.60, H: 6.14, N: 5.67

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 1.10(d,3H), 1.25(d,6H), 2.32(s,6H), 4.7-5.2(m,1H), 5.10(s,1H), 5.40(q,1H), 5.9-6.7(m,3H), 7.1-8.2(m,9H)

Example 24

1-methyl-3-furyl-3-propenyl methyl 4-(3-nitrophenyl)-2-6-dimethyl-1,4-dihydropyridine-3,5-dicarboxylate

Elemental Analysis; $C_{24}H_{24}N_2O_7$

Calcd. (%) C: 63.71, H: 5.35, N: 6.19

Found (%) C: 63.87, H: 5.24, N: 6.11

m.p.; Oily

IR ($cm^{-1}$); $\nu_{NH}$ 3330, $\nu_{CO}$ 1680, $\nu_{NO_2}$ 1530, 1350

NMR $\delta_{CDCl_3}$; 1.33(dd,3H), 2.33(s,6H), 3.62(s,3H), 5.11(s,1H), 5.23-5.73(m,1H), 5.7-6.6(m,5H), 7.2-8.2(m,5H)

Example 25

1-methyl-3-phenyl-2-propenyl 2-methoxyethyl 4-(3-nitrophenyl)-2,6-dimethyl-1,4-dihydropyridine-3,5-dicarboxylate

Elemental Analysis; $C_{28}H_{30}N_2O_7$

Calcd. (%) C: 66.39, H: 5.97, N: 5.53

Found (%) C: 66.44, H: 5.92, N: 5.47

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 1.30(d,3H), 2.30(s,6H), 3.29(d,3H), 3.48(t,2H), 4.15(t,2H), 5.10(s,1H), 5.50(q,1H), 5.9-6.7(m,3H), 7.0-8.2(m,9H)

Example 26

2,3-diphenyl-2-propenyl methyl 4-(3-nitrophenyl)-2,6-dimethyl-1,4-dihydropyridine-3,5-dicarboxylate

Elemental Analysis; $C_{31}H_{28}N_2O_6$

Calcd. (%) C: 70.98, H: 5.38, N: 5.34

Found (%) C: 71.05, H: 5.37, N: 5.30

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 2.23(s,3H), 2.29(s,3H), 3.60(s,3H), 4.89(s,2H), 5.02(s,1H), 6.0-6.6(m,3H), 7.0-8.1(m,10H)

Example 27

1-methyl-3-thienyl-2-propenyl methyl 4-(3-nitrophenyl)-2,6-dimethyl-1,4-dihydropyridine-3,5-dicarboxylate

Elemental Analysis; $C_{24}H_{24}N_2O_6S$

Calcd. (%) C: 61.53, H: 5.19, N: 5.98

Found (%) C: 61.55, H: 5.08, N: 5.79

m.p.; Oily

IR ($cm^{-1}$); $\nu_{NH}$ 3330, $\nu_{CO}$ 1680, $\nu_{NO_2}$ 1525, 1345

NMR $\delta_{CDCl_3}$; 1.37(dd,3H), 2.33(s,6H), 3.62(s,3H), 5.11(s,1H), 5.23-5.76(m,1H), 6.48(s,1H), 5.7-8.2(m,9H)

Example 28

Cinnamyl methyl 4-(2-furyl)-2,6-dimethyl-1,4-dihydropyridine-3,5-dicarboxylate

Elemental Analysis; $C_{23}H_{23}NO_5$

Calcd. (%) C: 70.21, H: 5.89, N: 3.56

Found (%) C: 70.27, H: 5.84, N: 3.50

m.p.; Oily

IR ($cm^{-1}$); $\nu_{NH}$ 3340, $\nu_{CO}$ 1690

NMR $\delta_{CDCl_3}$; 2.28(s,6H), 3.65(s,3H), 4.75(dd,2H), 5.23(s,1H), 5.88-6.60(m,5H), 7.1-7.4(m,6H)

Example 29

Cinnamyl methyl 4-(1-naphthyl)-2,6-dimethyl-1,4-dihydropyridine-3,5-dicarboxylate

Elemental Analysis; $C_{29}H_{27}NO_4$

Calcd. (%) C: 76.80, H: 6.00, N: 3.09

Found (%) C: 76.92, H: 5.88, N: 3.01

m.p.; Oily

IR ($cm^{-1}$); $\nu_{NH}$ 3330, $\nu_{CO}$ 1690

NMR $\delta_{CDCl_3}$; 2.23(ss,6H), 3.54(s,3H), 4.65(m,2H), 5.20(s,1H), 6.0-6.6(m,3H), 7.0-7.8(m,12H)

Example 30

4,4-diphenyl-3-butenyl methyl 4-(3-nitrophenyl)-2,6-dimethyl-1,4-dihydropyridine-3,5-dicarboxylate

Elemental Analysis; $C_{32}H_{30}N_2O_6$

Calcd. (%) C: 71.36, H: 5.61, N: 5.20

Found (%) C: 71.42, H: 5.53, N: 5.12

m.p.; Oily

IR ($cm^{-1}$); $\nu_{NH}$ 3330, $\nu_{CO}$ 1690, $\nu_{NO_2}$ 1530, 1350

NMR $\delta_{CDCl_3}$; 2.31(s,6H), 2.48(m,2H), 3.59(s,3H), 4.10(t,2H), 5.10(s,1H), 5.83(t,1H), 6.18(s,1H), 7.03-8.10(m,14H)

Example 31

Cinnamyl methyl 4-(2-fluorophenyl)-2,6-dimethyl-1,4-dihydropyridine-3,5-dicarboxylate

Elemental Analysis; $C_{25}H_{24}FNO_4$

Calcd. (%) C: 71.25, H: 5.74, N: 3.32

Found (%) C: 71.29, H: 5.70, N: 3.28

m.p.; 73.9-89.5°C

IR ($cm^{-1}$); $\nu_{NH}$ 3340, $\nu_{CO}$ 1695

NMR $\delta_{CDCl_3}$; 2.62(s,6H), 3.56(s,3H), 4.5-4.8(m,2H), 5.28(s,1H), 6.14(s,1H), 6.0-7.7(m,11H)

Example 32

434 mg (1 mM) of 4-(3-nitrophenyl)-2,6-dimethyl-1,4-dihydropyridine-3,5-dicarboxylic acid monocinnamyl ester, 226 mg (1.1 mM) of dicyclohexylcarbodiimide, 122 mg (1 mM) of 4-dimethylaminopyridine and 127 mg (1.2 mM) of 2-ethylthioethanol were dissolved in 10 ml of dichloroethene, and refluxed for 2 hours. After the reaction mixture was chilled, insoluble matters were filtered off. The solvent was evaporated under reduced pressure, and the residue was purified by silica gel column chromatography to obtain 511 mg of cinnamyl 2-ethylthioethyl 4-(3-nitrophenyl)-2,6-dimethyl-1,4-dihydropyridine-3,5-dicarboxylate (yield 98%).

Elemental Analysis; $C_{28}H_{30}N_2O_6S$

Calcd. (%) C: 64.35, H: 5.79, N: 5.36

Found (%) C: 64.48, H: 5.62, N: 5.11

m.p.; 142.5-143.5°C

IR ($cm^{-1}$); $\nu_{NH}$ 3320, $\nu_{CO}$ 1695, 1650, $\nu_{NO_2}$ 1530, 1350

NMR $\delta_{CDCl_3}$; 1.18(t,3H), 2.32(s,6H), 2.52(t,2H), 2.62(q,2H), 4.14(t,2H), 4.68(d,2H), 5.12(s,1H), 5.9-6.7(m,3H), 7.1-8.2(m,9H)

Examples 33-38

The following 1,4-dihydropyridine derivatives were prepared in the same manner as Example 32, except that 2-ethylthioethanol was replaced by the appropriate specified alcohol.

Example 33 Cinnamyl 2-phenylthioethyl 4-(3-nitrophenyl)-2,6-dimethyl-1,4-dihydropyridine-3,5-dicarboxylate

2-phenylthioethanol 185 mg (1.2 mM)

Yield; 554 mg (97%)

Elemental Analysis; $C_{32}H_{30}N_2O_6S$

Calcd. (%) C: 67.35, H: 5.30, N: 4.91

Found (%) C: 67.08, H: 5.51, N: 4.77

m.p.; 120.5-121.5°C

IR ($cm^{-1}$); $\nu_{NH}$ 3380, $\nu_{CO}$ 1705, 1640, $\nu_{NO_2}$ 1530, 1350

NMR $\delta_{CDCl_3}$; 2.32(s,6H), 3.04(t,2H), 4.20(t,2H), 4.18(d,2H), 5.12(s,1H), 5.9-6.7(m,3H), 7.2-8.2(m,14H)

Example 34

Cinnamyl 2-phenoxyethyl 4-(3-nitrophenyl)-2,6-dimethyl-1,4-dihydropyridine-3,5-dicarboxylate

2-phenoxyethanol 166 mg (1.2 mM)

Yield; 544 mg (98%)

Elemental Analysis; $C_{32}H_{30}N_2O_7$

Calcd. (%) C: 69.30, H: 5.41, N: 5.05

Found (%) C: 69.45, H: 5.27, N: 4.96

m.p.; Oily

IR ($cm^{-1}$); $\nu_{NH}$ 3340, $\nu_{CO}$ 1690, $\nu_{NO_2}$ 1530, 1350

NMR $\delta_{CDCl_3}$; 2.30(s,6H), 4.02(m,2H), 4.34(m,2H), 4.64(d,2H), 5.14(s,1H), 5.8-8.2(m,17H)

Example 35

Dicinnamyl 4-(3-nitrophenyl)-2,6-dimethyl-1,4-dihydropyridine-3,5-dicarboxylate

Cinnamyl alcohol 160 mg (1.2 mM)

Yield; 540 mg (98%)

Elemental Analysis; $C_{33}H_{30}N_2O_6$

Calcd. (%) C: 71.99, H: 5.49, N: 5.09

Found (%) C: 72.08, H: 5.35, N: 5.00

m.p.; 145-146°C

IR ($cm^{-1}$); $\nu_{NH}$ 3360, $\nu_{CO}$ 1690, 1640, $\nu_{NO_2}$ 1520, 1350

NMR $\delta_{CDCl_3}$; 2.30(s,6H), 4.66(d,4H), 5.15(s,1H), 5.8-6.6(m,5H), 7.0-8.2(m,14H)

Example 36

Cinnamyl phenylpropargyl 4-(3-nitrophenyl)-2,6-dimethyl-1,4-dihydropyridine-3,5-dicarboxylate

Phenylpropargyl alcohol 158 mg (1.2 mM)

Yield; 510 mg (93%)

Elemental Analysis; $C_{33}H_{28}N_2O_6$

Calcd. (%) C: 72.25, H: 5.14, N: 5.11

Found (%) C: 72.47, H: 5.06, N: 5.02

m.p.; 159.5-160.5°C

IR ($cm^{-1}$); $\nu_{NH}$ 3360, $\nu_{CO}$ 1690, 1640, $\nu_{NO_2}$ 1520, 1350

NMR $\delta_{CDCl_3}$; 2.43(s,6H), 4.68(d,2H), 4.83(s,2H), 5.15(s,1H), 5.8-6.7(m,3H), 7.1-8.2(m,14H)

Example 37

Cinnamyl 1-methyl-3-phenyl-2-propenyl 4-(3-nitrophenyl)-2,6-dimethyl-1,4-dihydropyridine-3,5-dicarboxylate

4-phenyl-3-buten-1-ol 178 mg (1.2 mM)

Yield; 559 mg (99%)

Elemental Analysis; $C_{34}H_{32}N_2O_6$

Calcd. (%) C: 72.32, H: 5.71, N: 4.96

Found (%) C: 72.51, H: 5.66, N: 4.92

m.p.; Oily

IR ($cm^{-1}$); $\nu_{NH}$ 3340, $\nu_{CO}$ 1690, $\nu_{NO_2}$ 1530, 1350

NMR $\delta_{CDCl_3}$; 1.35(d,3H), 2.30(s,6H), 4.66(d,2H), 5.12(s,1H), 5.42(m,1H), 5.8-6.7(m,5H), 7.1-8.2(m,14H)

Example 38

332 mg (1 mM) of 4-(3-nitrophenyl)-2,6-dimethyl-3-methoxycarbonyl-1,4-dihydropyridine-5-carboxylic acid, 226 mg (1.1 mM) of dicyclohexylcarbodiimide, 122 mg (1 mM) of 4-dimethylaminopyridine and 238 mg (1.2 mM) of 4-(1-naphthyl)-3-buten-1-ol were dissolved in 10 ml of dichloroethane, and refluxed for 2 hours. After the reaction mixture was chilled, insoluble matters were filtered off. The solvent was evaporated

under reduced pressure, and the residue was purified by silica gel chromatography to obtain 507 mg of methyl 4-(1-naphthyl)-3-butenyl 4-(3-nitrophenyl)-2,6-dimethyl-1,4-dihydropyridine-3,5-dicarboxylate (yield 99%).

Elemental Analysis; $C_{30}H_{28}N_2O_6$

Calcd. (%) C: 70.30, H: 5.51, N: 5.47

Found (%) C: 70.33, H: 5.44, N: 5.40

m.p.; Oily

IR ($cm^{-1}$); $\nu_{NH}$ 3360, $\nu_{CO}$ 1700, 1650, $\nu_{NO_2}$ 1535, 1350

NMR $\delta_{CDCl_3}$; 2.22(s,6H), 2.57(m,2H), 3.50(s,3H), 4.12(m,2H), 5.05(s,1H), 5.5-8.1(m,14H)

Examples 39-44

The following 1,4-dihydropyridine derivatives were prepared in the same manner as Example 38 except that 4-(1-naphthyl)-3-buten-1-ol was changed by the appropriate specific alcohol.

Example 39

1,1-dimethyl-4-phenyl-3-butenyl methyl 4-(3-nitrophenyl)-2,6-dimethyl-1,4-dihydropyridine-3,5-dicarboxylate

5-phenyl-2-methyl-4-penten-2-ol 211 mg (1.2 mM)

Yield; 147 mg (30%)

Elemental Analysis; $C_{28}H_{30}N_2O_6$

Calcd. (%) C: 68.56, H: 6.16, N: 5.71

Found (%) C: 68.77, H: 6.08, N: 5.55

m.p.; Oily

IR ($cm^{-1}$); $\nu_{NH}$ 3340, $\nu_{CO}$ 1690, $\nu_{NO_2}$ 1530, 1350

NMR $\delta_{CDCl_3}$; 1.42(s,6H), 2.29(s,6H), 2.64(d,2H), 3.56(s,3H), 5.02(s,1H), 5.6-6.5(m,3H), 7.0-8.1(m,9H)

Example 40

Methyl 5-(2-thienyl)-2,4-pentadienyl 4-(3-nitrophenyl)-2-6-dimethyl-1,4-dihydropyridine-3,5-dicarboxylate

5-(2-thienyl)-2,4-pentadien-1-ol 199 mg (1.2 mM)

Yield; 457 mg (95%)

Elemental Analysis; $C_{25}H_{24}N_2O_6S$

Calcd. (%) C: 62.49, H: 5.03, N: 5.83

Found (%) C: 62.63, H: 5.11, N: 5.77

m.p.; 122.8-154.7°C

IR ($cm^{-1}$); $\nu_{NH}$ 3300, $\nu_{CO}$ 1700, 1620, $\nu_{NO_2}$ 1470, 1355

NMR $\delta_{CDCl_3}$; 2.21(s,6H), 3.60(s,3H), 4.57(d,2H), 5.10(s,1H), 5.40-8.14(m,12H)

Example 41

Methyl 5-(2-furyl)-2,4-pentadienyl 4-(3-nitrophenyl)-2,6-dimethyl-1,4-dihydropyridine-3,5-dicarboxylate

5-(2-furyl)-2,4-pentadien-1-ol 180 mg (1.2 mM)

Yield; 446 mg (96%)

Elemental Analysis; $C_{25}H_{24}N_2O_7$

Calcd. (%) C: 64.65, H: 5.21, N: 6.03

Found (%) C: 64.81, H: 5.03, N: 5.99

m.p.; 112.2-113.7°C

IR ($cm^{-1}$); $\nu_{NH}$ 3300, $\nu_{CO}$ 1695, 1620, $\nu_{NO_2}$ 1465, 1350

NMR $\delta_{CDCl_3}$; 2.33(s,6H), 3.62(s,3H), 4.61(d,2H), 5.13(s,1H), 5.42-6.76(m,7H), 7.16-8.20(m,5H)

Example 42

Methyl 5-(2-naphthyl)-2,4-pentadienyl 4-(3-nitrophenyl)-2,6-dimethyl-1,4-dihydropyridine-3,5-dicarboxylate

5-(2-naphthyl)-2,4-pentadien-1-ol 252 mg (1.2 mM)

Yield; 488 mg (93%)

Elemental Analysis; $C_{30}H_{25}N_2O_6$

Calcd. (%) C: 70.72, H: 4.95, N: 5.50

Found (%) C: 70.90, H: 4.86, N: 5.44

m.p.; 181.4-187.6°C

IR ($cm^{-1}$); $\nu_{NH}$ 3300, $\nu_{CO}$ 1695, 1620, $\nu_{NO_2}$ 1460, 1350

NMR $\delta_{CDCl_3}$; 2.32(s,6H), 3.60(s,3H), 4.62(d,2H), 5.11(s,1H), 5.54-6.95(m,5H), 7.05-8.20(m,11H)

Example 43

Methyl 5-(1-naphthyl)-2,4-pentadienyl 4-(3-nitrophenyl)-2,6-dimethyl-1,4-dihydropyridine-3,5-dicarboxylate

5-(1-naphthyl)-2,4-pentadiene-1-ol 252 mg (1.2 mM)

Yield; 498 mg (95%)

Elemental Analysis; $C_{30}H_{25}N_2O_6$

Calcd. (%) C: 70.72, H: 4.95, N: 5.50

Found (%) C: 70.86, H: 4.89, N: 5.42

m.p.; 143.8-147.5°C

IR ($cm^{-1}$); $\nu_{NH}$ 3300, $\nu_{CO}$ 1695, 1620, $\nu_{NO_2}$ 1460, 1350

NMR $\delta_{CDCl_3}$; 2.29(s,3H), 2.33(s,3H), 3.60(s,3H), 4.63(d,2H), 5.16(s,1H), 5.53-8.43(m,16H)

Example 44

Methyl (Z)-4-(2-naphthyl)-3-butenyl 4-(3-nitrophenyl)-2,6-dimethyl-1,4-dihydropyridine-3,5-dicarboxylate (cis)

(Z)-4-(2-naphthyl)-3-buten-1-ol 238 mg (1.2 mM)

Yield; 481 mg (94%)

Elemental Analysis; $C_{30}H_{28}N_2O_6$

Calcd. (%) C: 70.30, H: 5.15, N: 5.47

Found (%) C: 70.41, H: 5.03, N: 5.41

m.p.; 139-140°C

IR ($cm^{-1}$); $\nu_{NH}$ 3360, $\nu_{CO}$ 1700, 1650, $\nu_{NO_2}$ 1530, 1350

NMR $\delta_{CDCl_3}$; 2.28(s,6H), 2.75(m,2H), 3.56(s,3H), 4.14(t,2H), 5.02(s,1H), 5.60(tt,1H), 6.25(s,1H), 6.60(d,1H), 7.1-8.2(m,11H)

Example 45

376 mg (1 mM) of 4-(3-nitrophenyl)-2,6-dimethyl-1,4-dihydropyridine-3,5-dicarboxylic acid mono 2-methoxyethyl ester, 226 mg (1.1 (mM) of dicyclohexylcarbodiimide, 122 mg (1 mM) of 4-dimethylaminopyridine and 192 mg (1.2 mM) of 5-phenyl-2,4-pentadien-1-ol were dissolved in 10 ml of dichloroethane, and refluxed for 2 hours. Thereafter, the reaction mixture was treated in the same manner as Example 39, and 503 mg of 2-methoxyethyl 5-phenyl-2,4-pentadienyl 4-(3-nitrophenyl)-2,6-dimethyl-1,4-dihydropyridine-3,5-dicarboxylate were obtained (yield 97%).

Elemental Analysis; $C_{29}H_{29}N_2O_7$

Calcd. (%) C: 67.30, H: 5.65, N: 5.41

Found (%) C: 67.41, H: 5.60, N: 5.29

m.p.; Oily

IR ($cm^{-1}$) $\nu_{NH}$ 3300, $\nu_{CO}$ 1695, 1620, $\nu_{NO_2}$ 1465, 1355

NMR $\delta_{CDCl_3}$; 2.34(s,6H), 3.29(s,3H), 3.37-3.70 (m,2H), 4.05-4.30(m,2H), 4.49(d,2H), 5.12 (s,1H), 5.50-6.72(m,5H), 7.08-8.16 (m,9H)

| | |
|---|---|
| m.p.; | Oily |
| IR ($cm^{-1}$); | $\nu_{NH}$ 3300, $\nu_{CO}$ 1695, 1620, $\nu_{NO_2}$ 1465, 1355 |
| NMR $\delta_{CDCl_3}$; | 2.34(s,6H), 3.29(s,3H), 3.37-3.70(m,2H), 4.05-4.30(m,2H), 4.49(d,2H), 5.12(s,1H), 5.50-6.72(m,5H), 7.08-8.16(m,9H) |

Example 46

Bis(5-phenyl-2-4-pentadienyl 4-(3-nitrophenyl)-2,6-dimethyl-1,4-dihydropyridine-3,5-dicarboxylate

4-(3-nitrophenyl)-2,6-dimethyl-1,4-dihydropyridine-3,5-dicarboxylic acid 3-(5-phenyl-2,4-pentadienyl)

ester 460 mg (1 mM)

Yield; 567 mg (94%)

Elemental Analysis; $C_{37}H_{34}N_2O_6$

Calcd. (%) C: 73.74, H: 5.69, N: 4.65

Found (%) C: 73.88, H: 5.60, N: 4.59

| | |
|---|---|
| m.p.; | 152.8-154.3°C |
| IR ($cm^{-1}$); | $\nu_{NH}$ 3320, $\nu_{CO}$ 1695, 1615, $\nu_{NO_2}$ 1465, 1350 |
| NMR $\delta_{CDCl_3}$; | 2.29(s,6H), 4.08(d,4H), 5.16(s,1H), 5.37-6.82(m,9H), 6.87-8.20(m,14H) |

Example 47

Cinnamyl methyl 4-(2-pyridyl)-2,6-dimethyl-1,4-dihydropyridine-3,5-dicarboxylate

4-(2-pyridyl)-2,6-dimethyl-3-methoxycarbonyl-1,4-dihydropyridine-5-carboxylic acid

294 mg (1 mM)

Cinnamyl alcohol 161 mg (1.2 mM)

Yield; 356 mg (88%)

Elemental Analysis; $C_{24}H_{24}N_2O_4$

Calcd. (%) C: 76.80, H: 6.00, N: 3.09

Found (%) C: 76.84, H: 5.92, N: 3.05

m.p.; Oily

IR ($cm^{-1}$); $\nu_{NH}$ 3330, $\nu_{CO}$ 1690

NMR $\delta_{CDCl_3}$; 2.22(s,3H), 2.25(s,3H), 3.56(s,3H), 4.65(dd,2H), 5.20(s,1H), 6.08-6.53(m,3H), 7.1-7.7(m,12H)

Example 48

Cinnamyl methyl 4-(2-thienyl)-2,6-dimethyl-1,4-dihydropyridine-3,5-dicarboxylate

4-(2-thienyl)-2,6-dimethyl-3-methoxycarbonyl-1,4-dihydropyridine-5-carboxylic acid

299 mg (1 mM)

Cinnamyl alcohol 161 mg (1.2 mM)

Yield; 319 mg (78%)

Elemental Analysis; $C_{23}H_{23}NO_4S$

Calcd. (%) C: 67.46, H: 5.66, N: 3.42

Found (%) C: 67.64, H: 5.32, N: 3.34

IR ($cm^{-1}$); $\nu_{NH}$ 3330, $\nu_{CO}$ 1690

NMR $\delta_{CDCl_3}$; 2.30(s,6H), 3.65(s,3H), 4.75(m,2H), 5.23(s,1H), 5.85-6.6(m,3H), 7.1-7.5(m,8H)

Example 49

664 mg (2 mM) of 4-(3-nitrophenyl)-2,6-dimethyl-1,4-dihydropyridine-3,5-dicarboxylic acid 3-methyl ester, 206 mg (2.1 mM) of 2,4-hexadien-1-ol, 433 mg (2.1 mM) of dicyclohexylcarbodiimide and 257 mg (2.1 mM) of 4-dimethylaminopyridine were dissolved in 20 ml of dichloroethane, and refluxed for 2 hours. Insoluble matters were filtered off, and the solvent was evaporated under reduced pressure. The residue was purified by silica gal chromatography, and 734 mg of methyl 2,4-hexadienyl 4-(3-nitrophenyl)-2,6-dimethyl-1,4-dihydropyridine-3,5-dicarboxylate were obtained (yield 89%).

Elemental Analysis; $C_{22}H_{24}N_2O_6$

Calcd. (%) C: 64.07, H: 5.87, N: 6.79

Found (%) C: 64.14, H: 5.82, N: 6.76

m.p.; 135-136.5°C

NMR $\delta_{CDCl_3}$; 1.72(d,3H), 2.34(s,6H), 3.62(s,3H), 4.52(d,2H), 5.3-6.4(m,5H), 7.2-8.1(m,4H)

Examples 50-54

The following 1,4-dihydropyridine derivatives were prepared in the same manner as Example 49 except that 4-(3-nitrophenyl)-2,6-dimethyl-1,4-dihydropyridine-3,5-dicarboxylic acid 3-methyl ester was replaced by the appropriate ester.

Example 50

Ethyl 2,4-hexadienyl 4-(3-nitrophenyl)-2,6-dimethyl-1,4-dihydropyridine-3,5-dicarboxylate

4-(3-nitrophenyl)-2,6-dimethyl-1,4-dihydropyridine-3,5-dicarboxylic acid

3-ethyl ester 692 mg (2 mM)

Yield; 734 mg (86.1%)

Elemental Analysis; $C_{23}H_{26}N_2O_6$

Calcd. (%) C: 64.78, H: 6.15, N: 6.57

Found (%) C: 64.89, H: 6.03, N: 6.76

m.p.; 114.7-115.6°C

NMR $\delta_{CDCl_3}$; 1.20(t,3H), 1.72(d,3H), 2.32(s,3H), 4.06(q,2H), 4.51(d,2H), 5.07(s,1H), 5.2-6.4(m,5H), 7.1-8.1(m,4H)

Example 51

Isopropyl 2,4-hexadienyl 4-(3-nitrophenyl)-2,6-dimethyl-1,4-dihydropyridine-3,5-dicarboxylate

4-(3-nitrophenyl)-2,6-dimethyl-1,4-dihydropyridine-3,5-dicarboxylic acid

3-isopropyl ester 721 mg (2 mM)

Yield; 801 mg (91%)

Elemental Analysis; $C_{24}H_{28}N_2O_6$

Calcd. (%) C: 65.44, H: 6.41, N: 6.36

Found (%) C: 65.61, H: 6.28, N: 6.26

m.p.; 102.8-104.3°C

NMR $\delta_{CDCl_3}$; 1.08(d,3H), 1.25(d,3H), 1.75(d,3H), 2.33(s,6H), 4.54(d,2H), 5.00(q,1H), 5.08(s,1H), 5.2-6.4(m,5H), 7.2-8.2(m,4H)

Example 52

2-methoxyethyl 2,4-hexadienyl 4-(3-nitrophenyl)-2,6-dimethyl-1,4-dihydropyridine-3,5-dicarboxylate

4-(3-nitrophenyl)-2,6-dimethyl-1,4-dihydropyridine-3,5-dicarboxylic acid

3-(2-methoxyethyl)ester 753 mg (2 mM)

Yield; 754 mg (82.6%)

Elemental Analysis;

Calcd. (%) C: 63.15, H: 6.18, N: 6.14

Found (%) C: 63.20, H: 5.98, N: 6.23

m.p.; 97.3-98.5°C

NMR $\delta_{CDCl_3}$; 1.71(d,3H), 2.32(s,6H), 3.51(t,2H), 4.16(t,2H), 4.50(d,2H), 5.10(s,1H), 5.2-6.4(m,5H), 7.1-8.2(m,4H)

Example 53

Cyclohexyl 2,4-hexadienyl 4-(3-nitrophenyl)-2,6-dimethyl-1,4-dihydropyridine-3,5-dicarboxylate

4-(3-nitrophenyl)-2,6-dimethyl-1,4-dihydropyridine-3,5-dicarboxylic acid

3-cyclohexyl ester 799 mg (2 mM)

Yield; 868 mg (90.3%)

Elemental Analysis; $C_{27}H_{32}N_2O_6$

Calcd. (%) C: 67.48, H: 6.71, N: 5.83

Found (%) C: 67.62, H: 6.49, N: 5.76

NMR $\delta_{CDCl_3}$; 0.9-2.1(m,10H), 1.73(d,3H), 2.31(s,6H), 4.4-4.9(m,1H), 4.51(d,2H), 5.08(s,1H), 5.2-6.4(m,4H), 6.77(s,1H), 7.1-8.2(m,4H)

Example 54

Cinnamyl 2,4-hexadienyl 4-(3-nitrophenyl)-2,6-dimethyl-1,4-dihydropyridine-3,5-dicarboxylate

4-(3-nitrophenyl)-2,6-dimethyl-1,4-dihydropyridine-3,5-dicarboxylic acid

3-cinnamyl ester 868 mg (2 mM)

Yield; 926 mg (90%)

Elemental Analysis; $C_{30}H_{30}N_2O_6$

Calcd. (%) C: 70.02, H: 5.88, N: 5.44

Found (%) C: 70.21, H: 5.68, N: 5.26

m.p.; 141-142°C

NMR $\delta_{CDCl_3}$; 1.67(d,3H), 2.31(s,6H), 4.50(d,2H), 4.68(d,2H), 5.16(s,1H), 5.2-6.9(m,7H), 7.1-8.2(m,9H)

Example 55

398 mg (1 mM) of 4-(3-nitrophenyl)-2,6-dimethyl-1,4-dihydropyridine-3,5-dicarboxylic acid 3-(2,4-hexadienyl) ester, 108 mg (1.1 mM) of 2,4-hexadien-1-ol, 227 mg (1.1 mM) of dicyclohexylcarbodiimide and 134 mg (1.1 mM) of 4-dimethylaminopyridine were dissolved in 20 ml of dichloroethane, and refluxed for 2 hours. Insoluble matters were filtered off, and the solvent was evaporated under reduced pressure. The residue was purified by silica gel column chromatography, and 428 mg (89.4%) of bis(2,4-hexadienyl) 4-(3-nitrophenyl)-2,6-dimethyl-1,4-dihydropyridine-3,5-dicarboxylate were obtained (yield 89.4%).

Elemental Analysis; $C_{27}H_{30}N_2O_6$ .

Calcd. (%) C: 67.77, H: 6.32, N: 5.85

Found (%) C: 67.91, H: 6.19, N: 5.71

m.p.; 145.1-146.1°C

NMR $\delta_{CDCl_3}$; 1.71(d,6H), 2.30(s,6H), 4.51(d,4H), 5.1(s,1H), 5.2-6.4(m,8H), 6.60(s,1H), 7.2-8.2(m,4H)

Example 56

332 mg (1 mM) of 4-(3-nitrophenyl)-2,6-dimethyl-1,4-dihydropyridine-3,5-dicarboxylic acid 3-methyl ester, 213 mg (1.1 mM) of 4-(2,6,6-trimethyl-1-cyclohexen-1-yl)-3-buten-2-ol, 103 mg (1.1 mM) of dicyclohexylcarbodiimide and 124 mg (1.1 mM) of 4-dimethylaminopyridine were dissolved in 20 ml of dichloroethane, and refluxed for 2 hours. Insoluble matters were filtered off, and the solvent was evaporated under reduced pressure. The residue was purified by silica gel column chromatography, and 468 mg of oily methyl 2-{4-(2,6,6-trimethyl-1-cyclohexen-1-yl)-3-buten-2-yl}4-(3-nitrophenyl)-2,6-dimethyl-1,4-dihydropyridine-3,5-dicarboxylate were obtained (yield 92.1%).

Elemental Analysis; $C_{29}H_{36}N_2O_6$

Calcd. (%) C: 68.48, H: 7.13, N: 5.51

Found (%) C: 68.59, H: 6.99, N: 5.36

NMR $\delta_{CDCl_3}$; 0.68-2.30(m,9H), 0.95(s,3H), 0.98(s,3H), 1.63(s,3H), 2.34(s,6H), 3.62(s,3H), 5.09(s,1H), 5.20-6.25(m,3H), 6.30(s,1H), 7.10-8.16(m,4H)

Examples 57

199 mg (0.5 mM) of 4-(3-nitrophenyl)-2,6-dimethyl-1,4-dihydropyridine-3,5-dicarboxylic acid mono (2,4-hexadienyl) ester, 44 mg (0.6 mM) of tert-butanol, 113 mg (0.55 mM) of dicyclohexylcarbodiimide and 61 mg (0.5 mM) of 4-dimethylaminopyridine were dissolved in 5 ml of dichloroethane, and refluxed for 2 hours. After the reaction mixture was chilled, insoluble matters were filtered off. The solvent was evaporated under reduced pressure, and the residue wad purified by silica gel column chromatography to obtain 198 mg of a crude material. This material was recrystallized with methanol, and 166 mg of tert-butyl 2,4-hexadienyl 4-(3-nitrophenyl)-2,6-dimethyl-1,4-dihydropyridine-3,5-dicarboxylate were obtained (yield 73%).

Elemental Analysis; $C_{25}H_{30}N_2O_6$

Calcd. (%) C: 66.06, H: 6.65, N: 6.16

Found (%) C: 66.23, H: 6.48, N: 6.09

m.p.; 151.8-154.1°C

IR ($cm^{-1}$); $\nu_{NH}$ 3340, $\nu_{CO}$ 1700, $\nu_{NO_2}$ 1530, 1350

NMR $\delta_{CDCl_3}$; 1.40(s,9H) 1.72(d,3H)
2.30 (s,6H), 4.50 (d, 2H),
5.01 (s, 1H), 5.2-6.3 (m, 5H),
7.2-8.2 (m, 4H)

Examples 58-67

The following 1,4-dihydropyridine derivatives were prepared in the same manner as Example 57 except that tert. butanol was replaced by the appropriate alcohol.

Example 58

Isobutyl 2,4-hexadienyl 4-(3-nitrophenyl)-2,6-dimethyl-1,4-dihydropyridine-3,5-dicarboxylate

2-methyl-1-propanol 44 mg (0.6 mM)

Yield; 174 mg (76.6%)

Elemental Analysis; $C_{25}H_{30}N_2O_6$

Calcd. (%) C: 66.06, H: 6.65, N: 6.16

Found (%) C: 66.16, H: 6.59, N: 6.14

m.p.; 136.4-137.3°C

IR ($cm^{-1}$); $\nu_{NH}$ 3330, $\nu_{CO}$ 1705, 1695,
$\nu_{NO_2}$ 1535, 1350

NMR $\delta_{CDCl_3}$; 0.82(dd,6H), 1.71(d,3H), 1.6-2.0(m,1H),
2.33(s,6H), 3.82(d,2H), 4.55(d,2H),
5.13(s,1H), 5.2-6.3(m,4H), 6.57(bs,1H),
7.2-8.2(m,4H)

Example 59

2-methyl-2-propen-1-yl 2,4-hexadienyl 4-(3-nitrophenyl)-2,6-dimethyl-1,4-dihydropyridine-3,5-dicarboxylate

2-methyl-2-propen-1-ol 43 mg (0.6 mM)

Yield; 180 mg (79.6%)

Elemental Analysis; $C_{25}H_{28}N_2O_6$

Calcd. (%) C: 66.36, H: 6.24, N: 6.19

Found (%) C: 66.21, H: 6.11, N: 6.12

m.p.; 130.6-131.6°C

IR ($cm^{-1}$); $\nu_{NH}$ 3330, $\nu_{CO}$ 1705, 1695, $\nu_{NO_2}$ 1530, 1350

NMR $\delta_{CDCl_3}$; 1.63(s,3H), 1.71(d,3H), 2.33(s,6H), 4.47(s,2H), 4.55(d,2H), 4.80(s,2H), 5.16(s,1H), 5.2-6.4(m,4H), 6.63(bs,1H), 7.15-8.2(m,4H)

Example 60

2-butenyl 2,4-hexadienyl 4-(3-nitrophenyl)-2,6-dimethyl-1,4-dihydropyridine-3,5-dicarboxylate

Crotyl alcohol 43 mg (0.6 mM)

Yield; 156 mg (69.0%)

Elemental Analysis; $C_{25}H_{28}N_2O_6$

Calcd. (%) C: 66.36, H: 6.24, N: 6.19

Found (%) C: 66.53, H: 6.07, N: 6.12

m.p.; 114.8-116.9°C

IR ($cm^{-1}$); $\nu_{NH}$ 3340, $\nu_{CO}$ 1705, 1690, $\nu_{NO_2}$ 1535, 1355

NMR $\delta_{CDCl_3}$; 1.5-1.75(m,6H), 2.32(s,6H), 4.3-4.7(m,4H), 5.10(s,1H), 5.2-6.3(m,6H), 6.41(bs,1H), 7.15-8.2(m,4H)

Example 61

3-butenyl 2,4-hexadienyl 4-(3-nitrophenyl)-2,6-dimethyl-1,4-dihydropyridine-3,5-dicarboxylate

3-buten-1-ol 43 mg (0.6 mM)

Yield; 116 mg (51.3%)

Elemental Analysis; $C_{25}H_{28}N_2O_6$

Calcd. (%) C: 66.36, H: 6.24, N: 6.19

Found (%) C: 66.51, H: 6.17, N: 6.08

m.p.; 83-83.6°C

IR ($cm^{-1}$); $\nu_{NH}$ 3340, $\nu_{CO}$ 1705, 1690, $\nu_{NO_2}$ 1535, 1350

NMR $\delta_{CDCl_3}$; 1.70(d,3H), 2.31(m,8H), 4.10(t,2H), 4.53(d,2H), 4.8-6.3(m,8H), 6.64(bs,1H), 7.1-8.2(m,4H)

Example 62

Allyl 2,4-hexadienyl 4-(3-nitrophenyl)-2,6-dimethyl-1,4-dihydropyridine-3,5-dicarboxylate

Allyl alcohol 35 mg (0.6 mM)

Yield; 144 mg (65.7%)

Elemental Analysis; $C_{24}H_{26}N_2O_6$

Calcd. (%) C: 65.44, H: 6.41, N: 6.36

Found (%) C: 65.52, H: 6.36, N: 6.27

m.p.; 93.2-101.4°C

IR ($cm^{-1}$); $\nu_{NH}$ 3330, $\nu_{CO}$ 1700, 1690, $\nu_{NO_2}$ 1530, 1350

NMR $\delta_{CDCl_3}$; 1.75(d,3H), 2.35(s,6H), 4.58(d,2H), 4.95-6.5(m,8H), 6.90(bs,1H), 7.2-8.25(m,4H)

Example 63

Benzyl 2,4-hexadienyl 4-(2-furyl)-2,6-dimethyl-1,4-dihydropyridine-3,5-dicarboxylate

Benzyl alcohol 65 mg

Recrystallized with acetonitrile instead of methanol

Yield; 224 mg (91.7%)

Elemental Analysis; $C_{28}H_{28}N_2O_6$

Calcd. (%) C: 68.84, H: 5.78, N: 5.73

Found (%) C: 69.03, H: 5.72, N: 5.64

m.p.; 173.3-175.2°C

IR ($cm^{-1}$); $\nu_{NH}$ 3330, $\nu_{CO}$ 1705, 1690, $\nu_{NO_2}$ 1530, 1355

NMR $\delta_{CDCl_3}$; 1.70(d,3H), 2.27(s,6H), 4.35-4.7(m,2H), 5.03(d,2H), 5.13(s,1H), 5.2-6.4(m,4H), 6.86(bs,1H), 7.0-8.15(m,9H)

Example 64

1-methyl-2-propenyl 2,4-hexadienyl 4-(3-nitrophenyl)-2,6-dimethyl-1,4-dihydropyridine-3,5-dicarboxylate

3-buten-2-ol 43 mg

Recrystallization was not carried out.

Yield; 236 mg (100%)

Elemental Analysis; $C_{25}H_{28}N_2O_6$

Calcd. (%) C: 66.36, H: 6.24, N: 6.19

Found (%) C: 66.57, H: 6.08, N: 6.24

m.p.; Oily

IR ($cm^{-1}$); $\nu_{NH}$ 3340, $\nu_{CO}$ 1700, 1690, $\nu_{NO_2}$ 1530, 1350

NMR $\delta_{CDCl_3}$; 1.26(dd,3H), 1.71(d,3H), 2.30(s,6H), 4.53(d,2H), 4.7-6.4(m,8H), 6.80(bs,1H), 7.2-8.2(m,4H)

Example 65

n-butyl 2,4-hexadienyl 4-(3-nitrophenyl)-2,6-dimethyl-1,4-dihydropyridine-3,5-dicarboxylate

n-butanol 44 mg

Yield; 203 mg (89.3%)

Elemental Analysis; $C_{25}H_{30}N_2O_6$

Calcd. (%) C: 66.06, H: 6.65, N: 6.16

Found (%) C: 66.17, H: 6.43, N: 6.07

m.p.; 105.5-106.7°C

IR ($cm^{-1}$); $\nu_{NH}$ 3340, $\nu_{CO}$ 1705, 1690, $\nu_{NO_2}$ 1530, 1350

NMR $\delta_{CDCl_3}$; 0.6-1.05(m,3H), 1.05-1.80(m,4H), 1.70(d,3H), 2.31(s,6H), 4.02(t,2H), 4.05(d,2H), 5.12(s,1H), 5.25-6.3(m,4H), 6.8-8.2(m,5H)

Example 66

n-propyl 2,4-hexadienyl 4-(3-nitrophenyl)-2,6-dimethyl-1,4-dihydropyridine-3,5-dicarboxylate

n-propanol 36 mg

Yield; 147 mg (66.7%)

Elemental Analysis; $C_{24}H_{28}N_2O_6$

Calcd. (%) C: 65.44, H: 6.41, N: 6.36

Found (%) C: 65.60, H: 6.27, N: 6.31

m.p.; 112.8-114.5°C

IR ($cm^{-1}$); $\nu_{NH}$ 3330, $\nu_{CO}$ 1705, 1690, $\nu_{NO_2}$ 1530, 1350

NMR $\delta_{CDCl_3}$; 0.84(t,3H), 1.52(m,2H), 2.33(s,6H), 3.99(t,2H), 4.53(d,2H), 5.12(s,1H), 5.2-6.4(m,4H), 6.55(bs,1H), 7.2-8.2(m,4H)

Example 67

Cyclopropylmethyl 2,4-hexadienyl 4-(3-nitrophenyl)-2,6-dimethyl-1,4-dihydropyridine-3,5-dicarboxylate

Cyclopropyl carbinol 43 mg

Yield; 86 mg (38%)

Elemental Analysis; $C_{25}H_{28}N_2O_6$

Calcd. (%) C: 66.36, H: 6.24, N: 6.19

Found (%) C: 66.28, H: 6.29, N: 6.12

m.p.; 119.6-120.8°C

IR ($cm^{-1}$); $\nu_{NH}$ 3340, $\nu_{CO}$ 1705, 1690, $\nu_{NO_2}$ 1535, 1350

NMR $\delta_{CDCl_3}$; 0.08-0.7(m,4H), 0.7-1.5(m,1H), 1.70(d,3H), 2.31(s,6H), 3.84(d,2H), 4.51(d,2H), 5.10(s,1H), 5.2-6.6(m,5H), 7.15-8.2(m,4H)

Example 68

Using 235 mg (2.1 mM) of 2,4-heptadien-1-ol instead of 2,4-hexadien-1-ol, methyl 2,4-heptadienyl 4-(3-nitrophenyl)-2,6-dimethyl-1,4-dihydropyridine-3,5-dicarboxylate was prepared in the same manner as Example 48.

Yield; 820 mg (96%)

Elemental Analysis; $C_{23}H_{26}N_2O_6$

Calcd. (%) C: 64.78, H: 6.15, N: 6.57

Found (%) C: 64.91, H: 6.02, N: 6.47

m.p.; Oily

IR ($cm^{-1}$); $\nu_{NH}$ 3330, $\nu_{CO}$ 1685, $\nu_{NO_2}$ 1530, 1350

NMR $\delta_{CDCl_3}$; 1.00(t,3H), 2.10(m,2H), 2.34(s,6H), 3.62(s,3H), 4.55(d,2H), 5.10(s,1H), 5.3-6.3(m,5H), 7.2-8.2(m,4H)

Example 69

Male spontaneously hypertensive rats (SHR) which had mean arterial blood pressures higher than 140 mmHg were used for the experiments. Arterial blood pressure was measured through a catheterized artery with a pressure transducer (MPU-0.5, manufactured by Nihon Kohden Kabushiki Kaisha). The catheter was inserted into the abdominal aorta via the median coccygeal artery of SHR under light ether anesthesia. The recordings were made on a rectigraph (manufactured by Sanei Sokki Kabushiki Kaisha).
1,4-dihydropyridine derivatives prepared in the foregoing examples were administered via a catheter which had been inserted previously into the caudal vein or duodenum at least two hours after the operation. SHR were restrained with wire mesh cage immediately after the operation. Each derivative to be tested was dissolved in 1.5% PVP/ethanol, and then diluted with 0.9% saline for intravenous administration (30 $\mu$g/kg), or suspended in 5% gum arabic solution for intraduodenal administration (1 mg/kg).

The results of the intravenous administration are shown in the following table.

Table 1

| Derivatives of the invention Ex.No. | ΔBP*1 mmHg | The time to reach the lowest BP min | Duration min |
|---|---|---|---|
| 3 | 55 | 70 | 90< |
| 5 | 87 | 25 | 180< |
| 6 | 12 | 13 | 240< |
| 7 | 72 | 12.5 | 90< |
| 8 | 60 | 1.6 | 100 |
| 9(trans) | 50 | 3.0 | 90< |
| 10 | 35 | 2.0 | 270< |
| 11 | 70 | 1.0 | 80< |
| 13 | 52.5 | 1.8 | 90 |
| 14 | 70 | 1.0 | 120< |
| 15 | 48 | 2.2 | - |
| 16 | 71 | 10 | 240< |
| 17 | 47 | 1.5 | - |
| 18 | 45 | 56 | 240< |
| 20 | 25 | 5.0 | - |
| 21 | 75 | 5.0 | 120< |
| 22 | 73 | 40 | 170< |
| 23 | 90 | 5 | 190< |
| 24 | 85 | 10.0 | 240< |
| 25 | 45 | 5 | 230< |
| 27 | 69 | 25.0 | 220< |
| 31 | 60 | 1.4 | 90< |
| 32 | 28 | 5 | 120< |
| 33 | 35 | 10 | 90< |
| 34 | 32 | 27 | 90< |
| 35 | 20 | 20 | - |
| 36 | 67 | 48 | 120< |
| 40 | 40 | 2.0 | 90< |
| 41 | 55 | 2.0 | 90< |
| 42 | 25 | 18 | 90 |
| 43 | 30 | 6.0 | 90< |
| 44 | 26.8 | 2.6 | - |

| | | | |
|---|---|---|---|
| 45 | 60 | 2.0 | 90< |
| 48 | 18 | 0.3 | - |
| 49 | 71.7 | 1.4 | (43.3)90< |
| 50 | 72 | 1.0 | (37) 90< |
| 51 | 60 | 1.4 | (13) 90< |
| 52 | 60 | 1.0 | 90< |
| 53 | 65 | 7.0 | 90< |
| 54 | 42 | 10 | 90< |
| 55 | 55 | 6.0 | 120< |
| 56 | 75 | 20 | 120< |
| 57 | 28 | 1 | - |
| 58 | 32 | 6 | - |
| 59 | 60 | 2 | - |
| 60 | 23 | 2.4 | - |
| 61 | 55 | 2.7 | - |
| 62 | 40 | 1.4 | - |
| 63 | 25 | 2.0 | - |
| 65 | 45 | 2.6 | - |
| 66 | 35 | 2 | - |
| 67 | 35 | 2.6 | - |
| A | 18.7 | 0.9 | 33.6±6.5 |
| B | 58.3 | 0.6 | 45-90 |

*1 Maximum depressor response

A Nifodipine

B Nicardipine

the number in parentheses indicates half-life.

The results of the intraduodenal administration are shown in the following table.

Table 2

| Derivatives of the invention Ex. No. | ΔBP*1 mmHg | The time to reach the lowest BP | half-life min. |
|---|---|---|---|
| 4 | 20 | 105 | 120< |
| 8 | 25 | 200 | 240 |
| 9 (trans) | 25 | 50 | 100 |
| 11 | 25 | 180 | 200< |
| 18 | 82 | 170 | 240< |
| 21 | 89 | 65 | 240< |
| 24 | 100 | 22 | 240< |
| 27 | 59 | 130 | 240< |
| 49 | 65 | 35 | 120< |
| A | 48.3 | 6.0 | 20.0 |
| B | 35 | 10.7 | 46.7 |
| C | 20 | 8 | 20.0 |
| D | 0 | - | - |
| E | 0 | - | - |

C: 4-(3-nitrophenyl)-2,6-dimethyl-1-4-dihydropyridine-3,5-dicarboxylic acid 3-methyl ester 5-allyl, ester

D: 4-(3-nitrophenyl)-2,6-dimethyl-1,4-dihydropyridine-3,5-dicarboxylic acid 3-methyl ester 5-propargyl ester

E: 4-(3-nitrophenyl)-2,6-dimethyl-1,4-dihydropyridine-3,5-dicarboxylic acid 3-methyl ester 5-(3-phenyl)-1-propyl ester

Acute toxicity of several derivatives was tested with male ddy mice weighed 15-30 grams by the up and down method. Each derivative was dissolved in 1.5% PVP/ethanol solution, and diluted with a saline solution. Such derivative solution was injected into the caudal vein, and the acute toxicity was determined.

The results are shown in the following Table.

Table 3

| Derivatives of the Invention Ex. No. | $LD_{50}$ mg/kg |
|---|---|
| 4 | 60< |
| 9 (trans) | 60< |
| 9 (cis) | 75< |
| 11 | 45 |
| A | 5 |
| B | 9.7 |

Example 70

Male SHR which had systolic blood pressure higher than 160 mmHg were used while conscious for the experiments. systolic blood pressure was measured by the tail cuff method with electrophygmomanometer (PE-300, made by Narco Co.). Each derivative (3 mg/kg) was administered orally, suspended in 5% gum arabic solution.

The results are shown in the following table.

Table 4

| Derivatives of the invention Ex. No. | BP mmHg | | Duration hr |
|---|---|---|---|
| | After 1 hour | After 3 hours | |
| 9 | - 30 | - 55 | 24< |
| 18 | -130 | -113 | 24< |
| 34 | - 30 | - 70 | 7-24 |
| A | - 45 | - 22.0 | < 7 |
| B | - 27 | - 4.5 | < 3 |

Example 71

Using both trans-form and cis-form of the 1,4-dihydropyridine derivatives prepared in Example 9, the depressor response of the rat was measured in the same manner as Example 69.

The results are shown in the following tables.

Table 5

| Derivatives of the Invention Ex. No. 9 | Dose µg/kg.i.v. | Depressure Response Max ΔBP mmHg | Peak min | Max ΔHR *1 beats/min | T 1/2 *2 min |
|---|---|---|---|---|---|
| trans | 10 | 21.7 | 2.8 | 43.3 | 11.3 |
| | 30 | 54.8 | 4.1 | 90.0 | 16.0 |
| | 100 | 72.5 | 4.3 | 80.0 | 37.5 |
| cis | 10 | 12.0 | 3.2 | 23.3 | 14.0 |
| | 30 | 26.8 | 2.6 | 62.5 | 15.0 |
| | 100 | 68.5 | 3.8 | 120 | 25.0 |

*1 Heart rates

*2 Half-life

Table 6

| Derivatives of the Invention Ex. No. 9 | $ED_{40}$ µg/kg i.v. |
|---|---|
| trans | 19.9 (y = 52.2x-27.8 r = 0.945) |
| cis | 40.8 (y = 54x-47.0 r = 0.918) |

## Claims

1. 1,4-dihydropyridine derivatives represented by the general formula

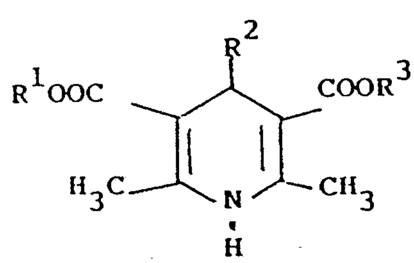

in which
$R^1$ represents a saturated or unsaturated, straight-chain, branched or cyclic hydrocarbyl group containing up to 6 carbon atoms, in which one carbon atom of the carbon skeleton thereof is optionally replaced by a oxygen or sulphur atom and/or in which one or more hydrogen atoms is/are optionally

substituted by phenyl, phenoxy or phenylthio;
$R^2$ represents phenyl, naphthyl or furyl in which one or more hydrogen atoms is are optionally substituted by a moiety selected from halogen atoms, alkylthio groups, nitro groups and cyano groups, and
$R^3$ represents (A), with $R^2$ being phenyl or substituted phenyl, a straight-chain or branched hydrocarbyl group containing two double bonds conjugated with each other, or (B) a group which is (a) an unsaturated straight-chain or branched aliphatic hydrocarbyl group containing from 3 to 6 carbon atoms, which group is optionally substituted by phenyl, an unsaturated carbon atom of said hydrocarbyl group having attached thereto a ring carbon atom of (b) a phenyl, naphthyl, furyl, thienyl, N-methyl-2-pyrrolyl or pyridyl group, with a said phenyl group optionally being substituted by a group selected from methyl, nitro and cyano groups,
excluding a compound which is cinnamyl methyl 4-(3-nitrophenyl)-2,6-dimethyl-1,4-dihydropyridine-3,5-dicarboxylate or methyl phenylpropargyl 4-(3-nitrophenyl)-2,6-dimethyl-1,4-dihydropyridine-3,5-dicarboxylate.

2. 1,4-dihydropyridine derivatives as claimed in claim 1, wherein $R^3$ (B)(a) is an unsaturated straight-chain hydrocarbyl group in which a hydrogen atom at the 1-position is substituted by $C_{1-4}$ alkyl or both hydrogen atoms at the 1-position are substituted by methyl groups.

3. 1,4-dihydropyridine derivatives as claimed in claim 1, wherein $R^2$ represents phenyl substituted by halogen, alkylthio, nitro, or cyano and $R^3$ represents a straight-chain, branched or cyclic hydrocarbon group containing two double bonds conjugated with each other.

4. 1,4-dihydropyridine derivatives represented by the general formula in which

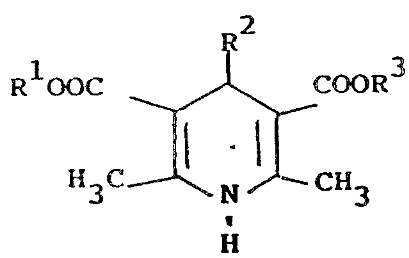

$R^1$ represents a saturated or unsaturated, straight-chain, branched or cyclic hydrocarbyl group containing up to 6 carbon atoms or an analogue thereof in which one or more carbon atoms of the carbon skeleton thereof is replaced by an oxygen atom or a sulphur atom and/or in which one or more hydrogen atoms is/are substituted by moieties selected from phenyl, phenoxy and phenylthio;
$R^2$ represents a phenyl, naphthyl, furyl, pyridyl or thienyl group in which one or more hydrogen atoms is/are optionally substituted by a moiety selected from halogen atoms, alkylthio groups, nitro groups and cyano groups, and
$R^3$ represents either (A) a group which is (a) an unsaturated straight-chain hydrocarbyl group containing 3 to 6 carbon atoms and in which a carbon atom of an unsaturated group is essentially bound by a direct chemical bond to an unsaturated carbon atom of (b) an unsaturated hydrocarbyl group containing up to 6 carbon atoms such that an unsaturated double bond of (a) is in conjunction with an unsaturated double bond of (b), or (B) a group which is (c) a said unsaturated straight-chain hydrocarbyl group (a) or a said unsaturated straight-chain hydrocarbyl group (a) which is phenyl substituted or in which a hydrogen atom at the 1-position is substituted by $C_{1-4}$ alkyl or both hydrogen atoms at the 1-position are substituted by methyl groups, an unsaturated carbon atom of said unsaturated straight-chain hydrocarbyl group having attached thereto a ring carbon atom of (d) a phenyl, naphthyl, furyl, thienyl, N-methyl-2-pyrrolyl or pyridyl group, with said phenyl group being
optionally substituted by a moiety selected from methyl, cyano and nitro groups,
excluding a compound which is cinnamyl methyl 4-(3-nitrophenyl)-2, 6-dimethyl-1,4-dihydropyridine-3,5-dicarboxylate or methyl phenylpropargyl (or 2-thienylpropargyl or 2-pyridylpropargyl) 4-(3-nitrophenyl)-2,6-dimethyl-1,4-dihydropyridine-3,5-dicarboxylate.

**5.** A pharmacologically acceptable acid addition salt of a compound as claimed in any preceding claim.

**6.** A 1,4-dihydropyridine derivative as claimed in any preceding claim, which is in the form of a cis- or a trans-isomer.

**7.** A 1,4-dihydropyridine derivative as claimed in any preceding claim, wherein in $R^1$, one or more carbon atoms of a carbon chain or carbocyclic ring, is/are replaced by oxygen atom(s) and/or one or more hydrogen atoms is/are substituted by phenyl.

**8.** The compound methyl 2-{4-(2,6,6-trimethyl-1-cyclohexen-1-yl)-3-buten-2-yl}4-(3-nitrophenyl)-2,6-dimethyl-1,4-dihydropyridine-3,5-dicarboxylate.

**9.** The compound cinnamyl 2-methoxyethyl 4-(3-nitrophenyl)-2,6-dimethyl-1,4-dihydropyridine-3,5-dicarboxylate.

**10.** The compound 4-β-naphthyl-3-butenyl methyl 4-(3-nitrophenyl)-2,6-dimethyl-1,4-dihydropyridine-3,5-dicarboxylate. (trans or cis).

**11.** A method for the production of a 1,4-dihydropyridine derivative as claimed in claim 1 or 4, which comprises carrying out a ring closing reaction involving

(1) a compound of general formula

```
      O       O
      "       "
CH3-C-CH2-C-OR^x
```

$$CH_3-C(=O)-CH_2-C(=O)-OR^x$$

a compound having the general formula

$R^2$-CHO

and a compound having the general formula

```
     NH2   O
      '    "
CH3-C=CH-C-OR^y
```

$$CH_3-C(NH_2)=CH-C(=O)-OR^y$$

(2) a compound of general formula

```
      O  O
      "  "
CH3-C-C-C-OR^x
        "
        CHR^2
```

$$CH_3-C(=O)-C(=CHR^2)-C(=O)-OR^x$$

and a compound of general formula

```
     NH2   O
      '    "
CH3-C=CH-C- OR^y; or
```

$$CH_3-C(NH_2)=CH-C(=O)-OR^y$$; or

(3) ammonia, a compound of general formula

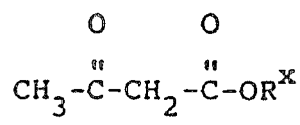

and a compound of general formula

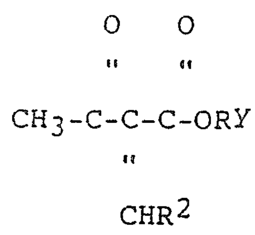

wherein $R^x$ and $R^y$ are $R^1$ and $R^3$ or $R^3$ and $R^1$ respectively, $R^1$, $R^2$ and $R^3$ being as defined in claim 1 or 4, reaction being carried out in the absence of a solvent active to the reaction.

**12.** A method for the production of a 1,4-dihydropyridine compound as claimed in claim 1, which comprises reacting a compound having the general formula

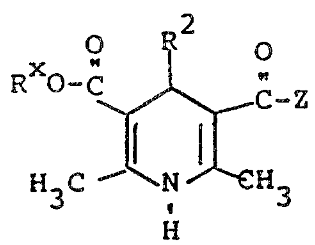

in which Z represents a hydroxyl group or an acid residue of an active ester with an alcohol of general formula $R^y$ -OH, $R^x$ and $R^y$ being $R^1$ and $R^3$ or $R^3$ and $R^1$ respectively, $R^1$, $R^2$ and $R^3$ being as defined in claim 1.

**13.** A pharmaceutical composition, which comprises a compound as claimed in any one of claims 1 to 10, in association with a pharmacologically acceptable adjuvant.

**Revendications**

**1.** Dérivés de 1,4-dihydropyridine représentés par la formule générale

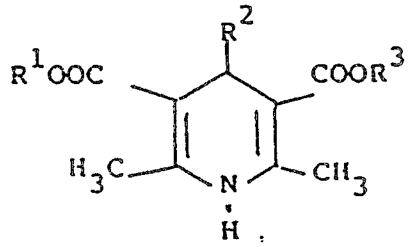

dans laquelle
$R^1$ représente un groupe hydrocarboné saturé ou insaturé, à chaîne linéaire, ramifiée ou cyclique,

contenant jusqu'à 6 atomes de carbone, dans lequel un atome de carbone de son squelette carboné est éventuellement remplacé par un atome d'oxygène ou de soufre et/ou dans lequel un ou plusieurs atomes d'hydrogène est/sont éventuellement remplacé(s) par un groupe phényle, phénoxy ou phénylthio ;

$R^2$ représente un groupe phényle, naphtyle ou furyle dans lequel un ou plusieurs atomes d'hydrogène est/sont éventuellement remplacé(s) par un fragment choisi parmi des atomes d'halogène, des groupes alkylthio, des groupes nitro et des groupes cyano, et

$R^3$ représente (A), $R^2$ étant un groupe phényle ou phényle substitué, un groupe hydrocarboné à chaîne linéaire ou ramifiée contenant 2 doubles liaisons conjuguées l'une avec l'autre, ou (B) un groupe qui est (a) un groupe hydrocarboné aliphatique à chaîne linéaire ou ramifiée, insaturé, contenant de 3 à 6 atomes de carbone, ce groupe étant éventuellement substitué par un groupe phényle, un atome de carbone insaturé dudit groupe hydrocarboné étant lié à un atome de carbone cyclique de (b) un groupe phényle, naphtyle, furyle, thiènyle, N-méthyl-2-pyrrolyle ou pyridyle, ledit groupe phényle étant éventuellement substitué par un groupe choisi parmi les groupes méthyle, nitro et cyano,

à l'exclusion d'un composé qui est le 4-(3-nitrophényl)-2,6--diméthyl-1,4-dihydropyridine-3,5-dicarboxylate de cinnamyle et de méthyle ou le 4-(3-nitrophényl)-2,6-diméthyl-1,4-dihydropyridine--3,5-dicarboxylatede méthyle et de phénylpropargyle.

2. Dérivés de 1,4-dihydropyridine tels que revendiqués dans la revendication 1, dans lesquels $R^3$ (B) (a) est un groupe hydrocarboné insaturé à chaîne linéaire dans lequel un atome d'hydrogène en position 1 est remplacé par un groupe alkyle en $C_{1-4}$ ou les deux atomes d'hydrogène en position 1 sont remplacés par des groupes méthyle.

3. Dérivés de 1,4-dihydropyridine tels que revendiqués dans la revendication 1, dans lesquels $R^2$ représente un groupe phényle substitué par halogène, alkylthio, nitro ou cyano et $R^3$ représente un groupe hydrocarboné à chaîne linéaire, ramifiée ou cyclique, contenant deux doubles liaisons conjuguées l'une avec l'autre.

4. Dérivés de 1,4-dihydropyridine représentés par la formule générale

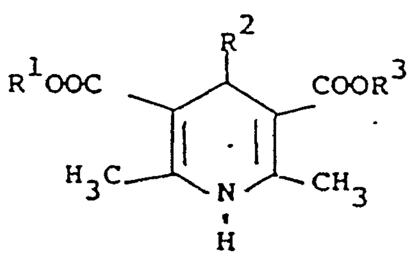

dans laquelle

$R^1$ représente un groupe hydrocarboné, saturé ou insaturé, à chaîne linéaire, ramifiée ou cyclique, contenant jusqu'à 6 atomes de carbone, ou un de ses analogues dans lequel un ou plusieurs atomes de carbone de son squelette carboné est remplacé par un atome d'oxygène ou un atome de soufre et/ou dans lequel un ou plusieurs atomes d'hydrogène est/sont remplacé(s) par des fragments choisis parmi les groupes phényle, phénoxy et phénylthio ;

$R^2$ représente un groupe phényle, naphtyle, furyle, pyridyle ou thiènyle dans lequel un ou plusieurs atomes d'hydrogène est/sont éventuellement remplacé(s) par un fragment choisi parmi les atomes d'halogène, les groupes alkylthio, les groupes nitro et les groupes cyano, et

$R^3$ représente soit (A) un groupe qui est (a) un groupe hydrocarboné insaturé, à chaîne linéaire, contenant 3 à 6 atomes de carbone et dans lequel un atome de carbone d'un groupe insaturé est principalement lié, par une liaision chimique directe, à un atome de carbone insaturé de (b) un groupe hydrocarboné insaturé contenant jusqu'à 6 atomes de carbone de telle sorte qu'une double liaison insaturée de (a) est conjuguée à une double liaison insaturée de (b), soit (B) un groupe qui est (c) un groupe hydrocarboné insaturé à chaîne linéaire (a) ou un dit groupe hydrocarboné insaturé à chaîne linéaire (a) qui est substitué par le phényle ou dans lequel un atome d'hydrogène en position 1 est remplacé par un groupe alkyle en $C_{1-4}$ ou les deux atomes d'hydrogène en position 1 sont remplacés par des groupes méthyle, un atome de carbone insaturé dudit groupe hydrocarboné insaturé, à chaîne

linéaire, étant lié à - un atome de carbone cyclique de (d) un groupe phényle, naphtyle, furyle, thiènyle, N-méthyl-2-pyrrolyle ou pyridyle, ledit groupe phényle étant éventuellement substitué par un fragment choisi parmi les groupes méthyle, cyano et nitro,
à l'exclusion d'un composé qui est le 4-(3-nitrophényl)-2,6--diméthyl-1,4-dihydropyridine-3,5-dicarboxylate de cinnamyle et de méthyle ou le 4-(3-nitrophényl)-2,6-diméthyl-1,4-dihydropyridine--3,5-dicarboxylate de méthyle et de phénylpropargyle (ou 2-thiènylpropargyle ou 2-pyridylpropargyle).

**5.** Sel d'addition d'acide , pharmacologiquement acceptable, d'un composé selon l'une quelconque des revendications précédentes.

**6.** Dérivé de 1,4-dihydropyridine selon l'une quelconque des revendications précédentes qui se présente sous la forme d'un isomère cis ou trans.

**7.** Dérivé de 1,4-dihydropyridine selon l'une quelconque des revendications précédentes, dans lequel dans $R^1$, un ou plusieurs atomes de carbone d'une chaîne carbonée ou d'un noyau carbocyclique, est/sont remplacé(s) par un ou des atome(s) d'oxygène et/ou un ou plusieurs atome(s) d'hydrogène est/sont remplacé(s) par le groupe phényle.

**8.** Le composé qui est le 4-(3-nitrophényl)-2,6-diméthyl--1,4-dihydropyridine-3,5-dicarboxylatede méthyle et de 2-(4-(2,6,6--triméthyl-1-cyclohexèn-1-yl)3-butèn-2-yle).

**9.** Le composé qui est le 4-(3-nitrophényl)-2,6-diméthyl--1,4-dihydropyridine-3,5-dicarboxylate de cinnamyle et de 2-méthoxyéthyle.

**10.** Le composé qui est le 4-(3-nitrophényl)-2,6-diméthyl-1,4-dihydropyridine-3,5-dicarboxylate de 4-beta-naphtyl-3-butényle et de méthyle (trans ou cis).

**11.** Procédé pour la production d'un dérivé de 1,4-dihydropyridine selon la revendication 1 ou 4, qui consiste à réaliser une réaction de cyclisation impliquant
(1) un composé de formule générale

$$CH_3-\overset{\overset{O}{\|}}{C}-CH_2-\overset{\overset{O}{\|}}{C}-OR^x$$

un composé ayant la formule générale

$R^2$-CHO

et un composé ayant la formule générale

$$CH_3-\overset{\overset{NH_2}{|}}{C}=CH-\overset{\overset{O}{\|}}{C}-OR^y$$

(2) un composé de formule générale

O O
" "
$CH_3-C-C-C-OR^x$
"
$CHR^2$

et un composé de formule générale

$NH_2$ O
' "
$CH_3-C=CH-C-OR^y$; ou

(3) l'ammoniac, un composé de formule générale

O O
" "
$CH_3-C-CH_2-C-OR^x$

et un composé de formule générale

O O
" "
$CH_3-C-C-C-OR^y$
"
$CHR_2$

dans lesquelles $R^x$ et $R^y$ sont respectivement $R^1$ et $R^3$ ou $R^3$ et $R^1$, $R^1$, $R^2$ et $R^3$ étant tels que définis dans la revendication 1 ou 4, la réaction étant effectuée en l'absence d'un solvant actif vis-à-vis de la réaction.

**12.** Procédé pour la production d'un dérivé de 1,4-dihydropyridine selon la revendication 1, qui comprend la réaction d'un composé ayant la formule générale

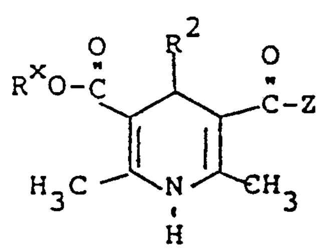

dans laquelle Z représente un groupe hydroxyle ou un résidu acide d'un ester actif, avec un alcool de formule générale $R^y$-OH, $R^x$ et $R^y$ étant respectivement $R^1$ et $R^3$ ou $R^3$ et $R^1$, $R^1$, $R^2$ et $R^3$ étant tels que définis dans la revendication 1.

**13.** Composition pharmaceutique, qui comprend un composé selon l'une quelconque des revendications 1

à 10, en association avec un adjuvant pharmacologiquement acceptable.

**Ansprüche**

**1.** 1,4-Dihydropyridinderivate gemäß der allgemeinen Formel

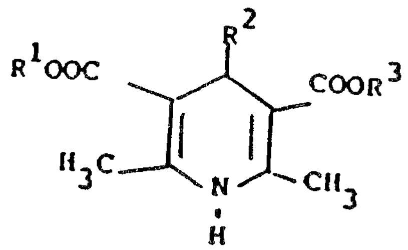

in der

- $R^1$ eine gesättigte oder ungesättigte, geradkettige, verzweigte oder cyclische Kohlenwasserstoffgruppe mit bis zu 6 C-Atomen bedeutet, in der ein oder mehrere der Kohlenstoffatome des Kohlenstoffskeletts ggf. auch durch ein Sauerstoff- oder Schwefelatom ersetzt ist und/oder eines oder mehrere der Wasserstoffatome fakultativ durch Phenyl, Phenoxy oder Phenylthio substituiert ist/sind,
- $R^2$ Phenyl, Naphthyl oder Furyl entspricht, wobei eines oder mehrere dar Wasserstoffatome ggf. auch durch eine Komponente ausgewählt aus Halogenatomen, Alkylthiogruppen, Nitrogruppen und Cyangruppen substituiert ist/sind, und
- $R^3$ entweder (A) eine geradkettige oder verzweigte Kohlenwasserstoffgruppe mit zwei miteinander konjugierten Doppelbindungen ist, wobei $R^2$ Phenyl oder substituiertes Phenyl ist, oder (B) eine Gruppe darstellt, die (a) ein ungesättigter geradkettiger oder verzweigter aliphatischer Kohlenwasserstoffrest mit 3 bis 6 C-Atomen, der auch durch Phenyl substituiert sein kann, ist und wobei an ein ungesättigtes Kohlenstoffatom des Kohlenwasserstoffrests auch ein Ringkohlenstoffatom von (b) einer Phenyl, Naphthyl, Furyl, Thienyl, N-Methyl-2-pyrrolyl oder Pyridyl-Gruppe oder einer durch Methyl, Nitro oder Cyan substituierten Phenylgruppe gebunden sein kann, mit Ausnahme der Verbindungen Cinnamylmethyl-4(3-nitrophenyl)-2,6-dimethyl-1,4-dihydropyridin-3,5-dicarboxylat oder Methylphenyl-progargyl-4-(3-Nitrophenyl)-2,6-dimethyl-1,4-dihydropyridin-3,5-dicarboxylat.

**2.** 1,4-Dihydropyridinderivate gemäß Anspruch 1, worin

- $R^3$ (B) (a) einer ungesättigten geradkettigen Kohlenwasserstoffgruppe entspricht, in der ein Wasserstoffatom in 1-Stellung durch ein $C_{1-4}$-Alkyl oder beide Wasserstoffatome in der 1-Stellung durch Methylgruppen ersetzt sind.

**3.** 1,4-Dihydropyridinderivate gemäß Anspruch 1, worin

- $R^2$ durch Halogen, Alkylthio, Nitro oder Cyan substituiertes Phenyl ist und $R^3$ eine geradkettige, verzweigte oder cyclische Kohlenwasserstoffgruppe mit zwei miteinander konjugierten Doppelbindungen darstellt.

**4.** 1,4-Dihydropyridinderivate gemäß der allgemeinen Formel

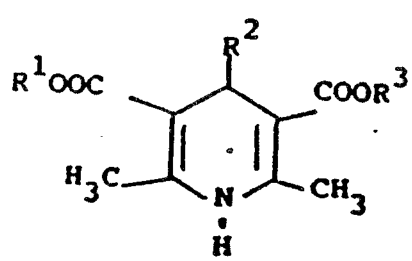

in der

- $R^1$ eine gesättigte oder ungesättigte, geradkettige, verzweigte oder cyclische Kohlenwasserstoffgruppe mit bis zu 6 C-Atomen bedeutet oder ein Analogon dazu ist, in dem eines oder mehrere der Kohlenstoffatome des Kohlenstoffskeletts durch ein Sauerstoffatom oder Schwefelatom ersetzt ist und/oder ein oder mehrere Wasserstoffatome durch Phenyl, Phenoxy und Phenylthio substituiert ist/sind,
- $R^2$ einer Phenyl, Naphthyl, Furyl, Pyridyl oder Thienyl-Gruppe entspricht, in der eines oder mehrere der Wasserstoffatome fakultativ durch eine Komponente ausgewählt aus Halogenatomen, Alkylthiogruppen, Nitrogruppen und Cyangruppen ersetzt ist/sind, und
- $R^3$ entweder (A) eine Gruppe bedeutet, die (a) einem ungesättigten geradkettigen Kohlenwasserstoffrest mit 3-6 C-Atomen entspricht und in dem ein Kohlenstoffatom der ungesättigten Gruppierung durch direkte chemische Bindung an eine ungesättigte Kohlenwasserstoffgruppe mit bis zu 6 C-Atomen gebunden ist, so daß eine ungesättigte Doppelbindung von (a) in Konjugation mit einer ungesättigten Doppelbindung in (b) steht, oder (B) eine Gruppe bedeutet, die (c) eine der ungesättigten geradkettigen Kohlenwasserstoffgruppen entsprechend (a) oder eine Substitutionsverbindungvon (a) ist mit einer Phenylgruppierung oder dem Ersatz eines Wasserstoffatoms in der 1-Stellung durch ein $C_{1-4}$-Alkyl oder von beiden Wasserstoffatomen in 1-Stelung durch Methylgruppen, und

  wobei ein ungesättigtes Kohlenstoffatom der ungesättigten geradkettigen Kohlenwasserstoffgruppe an ein Ringkohlenstoffatom von (d) einer Phenyl, Naphthyl, Furyl, Thienyl, N-Methyl-1-pyrrolyl oder Pyridyl-Gruppe gebunden ist und in der Phenylgruppe fakultativ eine Substitution mit Methyl, Cyan oder Nitro-Gruppen vorliegen kann, mit Ausnahme der Verbindungen Cinnamylmethyl-4(3-nitrophenyl)-2,6-dimethyl-1,4-dihydropyridin-3,5-dicarboxylat oder Methylphenyl-propargyl-(oder 2-Thienylpropargyl-oder 2-Pyridylprogargyl-)4-(3-nitrophenyl)-2,6-dimethyl-1,4-dihydropyridin-3,5-dicarboxylat.

**5.** Ein pharmakologisch akzeptables Säureanlagerungssalz von einer der in den vorangehenden Ansprüchen beanspruchten Verbindungen.

**6.** Ein 1,4-Dihydropyridinderivat gemäß einem der vorangehenden Ansprüche, vorliegend in der Form eines Cis- oder Trans-Isomeren.

**7.** Ein 1,4-Dihydropyridinderivat gemäß einem der vorangehenden Ansprüche, wobei in

- $R^1$ eines oder mehrere der Kohlenstoffatome in der Kohlenstoffkette oder im carbocyclischen Ring durch Sauerstoffatom(e) ersetzt ist/sind und eines oder mehrere der Wasserstoffatome durch Phenyl substituiert ist/sind.

**8.** Methyl-2-[4-(2,6,6-trimethyl-1-cyclohexen-1-yl)-3-buten-2-yl]4-(3-nitrophenyl)-2,6-dimethyl-1,4-dihydropyridin-3,5-dicarboxylat.

**9.** Cinnamyl-2-methoxyethyl-4-(3-nitrophenyl)-2,6-dimethyl-1,4-dihydropyridin-3,5-dicarboxylat.

**10.** 4-$\beta$-Naphthyl-3-butenylmethyl-4-(3-nitrophenyl)-2,6-dimethyl-1,4-dihydropyridin-3,5-dicarboxylat (trans oder cis).

**11.** Verfahren zur Herstellung von einem 1,4-Dihydropyridinderivat gemäß Anspruch 1 oder 4, welches umfaßt die Durchführung einer Ringschlußreaktion durch Zusammenbringen

(1) von einer Verbindung der allgemeinen Formel

$$CH_3-\overset{O}{\overset{\|}{C}}-CH_2\overset{O}{\overset{\|}{C}}-OR^x$$

einer Verbindung der allgemeinen Formel

$R^2$-CHO

und einer Verbindung der allgemeinen Formel

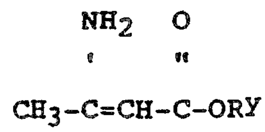

(2) von einer Verbindung der allgemeinen Formel

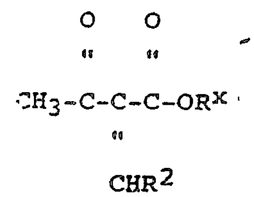

und einer Verbindung der allgemeinen Formel

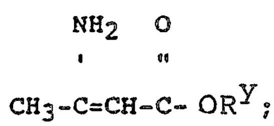 ; or

oder
(3) von Ammoniak, einer Verbindung der allgemeinen Formel

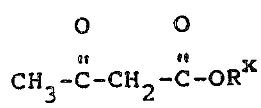

und einer Verbindung der allgemeinen Formel

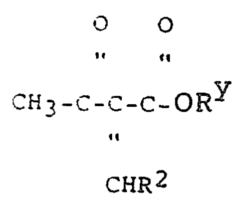

wobei $R^x$ und $R^y$ jeweils $R^1$ und $R^3$ oder $R^3$ und $R^1$ bedeuten,
sowie $R^1$, $R^2$ und $R^3$ der in den Ansprüchen 1 und 4 angeführten Definition entsprechen und die Reaktion in Abwesenheit eines aktiv in die Reaktion eingreifenden Lösungsmittels erfolgt.

**12.** Verfahren zur Herstellung von einer 1,4-Dihydropyridinderivatverbindung gemäß Anspruch 1, welches umfaßt die Umsetzung einer Verbindung gemäß der allgemeinen Formel

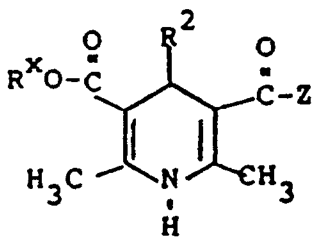

in der Z eine Hydroxylgruppe oder den Säurerest eines aktiven Esters darstellt, mit einem Alkohol der allgemeinen Formel

$R^yOH$

und wobei
$R^x$ und $R^y$ für $R^1$ und $R^3$ beziehungsweise für $R^3$ und $R^1$ stehen sowie
$R^1$, $R^2$ und $R^3$ der im Anspruch 1 angeführten Definition entsprechen.

**13.** Pharmazeutische Zusammensetzung, welche eine der in den Ansprüchen 1 bis 10 beanspruchten Verbindungen zusammen mit einem pharmakologisch akzeptablen Adjuvans enthält.

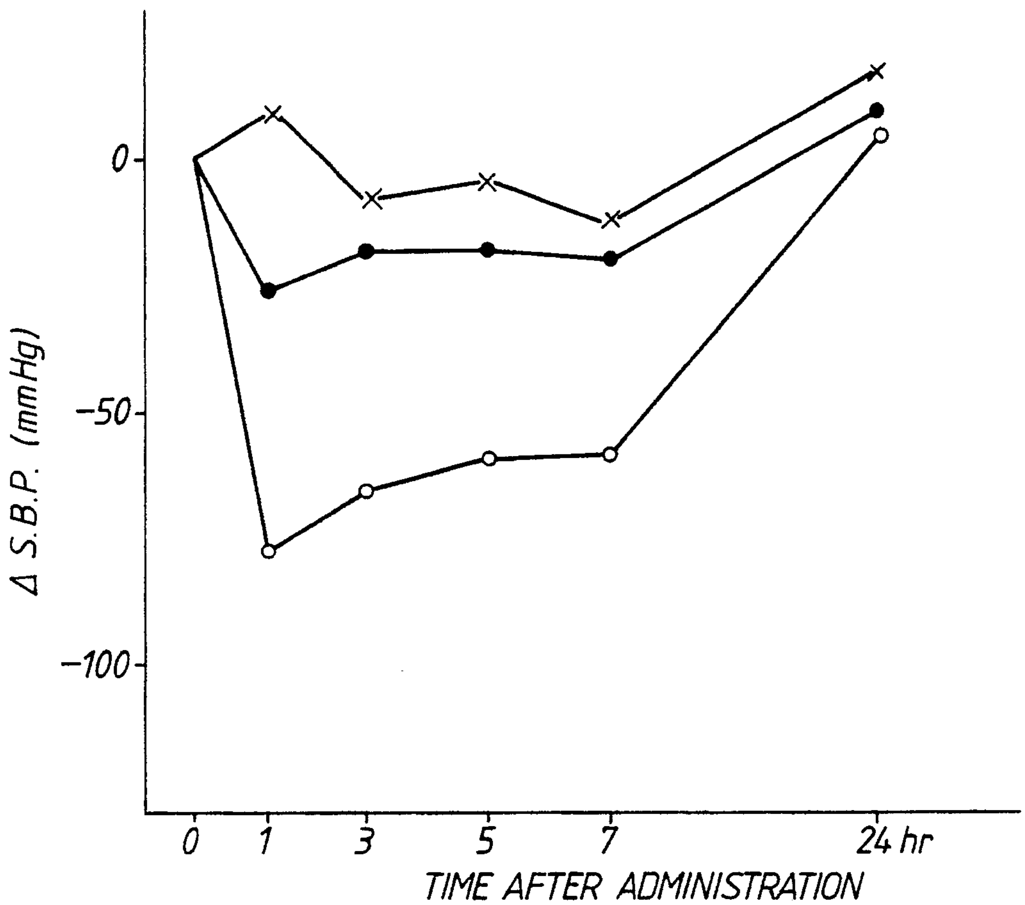
0
-50
-100
Δ S.B.P. (mmHg)
0
1
3
5
7
24 hr
TIME AFTER ADMINISTRATION